# EUROPEAN PATENT APPLICATION

(11) **EP 4 635 955 A2**
(43) Date of publication of application: **22.10.2025**
(21) Application number: 23902775.8
(22) Date of filing: 14.12.2023
(51) Int. Cl.: C07D 471/04, C07D 401/14, C07D 487/04, A61K 31/519, A61P 31/00, A61P 35/00

(54) **COMPOUND AND USE THEREOF**

(30) Priority: 16.12.2022 CN 202211621444
(71) Applicant: OnQuality Pharmaceuticals China Ltd., Shanghai 201112 (CN)
(72) Inventor: LIU, Shilan, Shanghai 200431 (CN); ZHANG, Shiyi, Shanghai 200431 (CN); ZHAO, Chengxu, Shanghai 200431 (CN)
(74) Representative: Elzaburu S.L.P.
(86) International application number: PCT/CN2023/138629
(87) International publication number: WO 2024/125581

(57) **Abstract**

The present application relates to a compound as a CDK4/6 inhibitor is delivered to the gastrointestinal trac in a targeted manner. The present application further relates to a pharmaceutical composition comprising the compound, a method of using the compound for treating, preventing, or ameliorating gastrointestinal side effects caused by chemotherapy, and a method and intermediates for preparing the compound.

## Description

### FIELD

The present application relates to the field of biomedicine, specifically relating to a CDK4 /6 inhibitor and use thereof.

### BACKGROUND

CDK (cyclin-dependent kinase) inhibitors have therapeutic potential for a wide range of diseases, including cancer, diabetes, kidney disease, neurodegenerative disorders, infectious diseases, tumors, and side effects of cancer therapy. However, orally administered systemic CDK inhibitors frequently cause adverse effects such as neutropenia, leukopenia, and anemia.

Gastrointestinal side effects caused by chemotherapy are common adverse reactions in cancer therapy, significantly affecting patients' survival and mortality rates. The gastrointestinal side effects caused by chemotherapy include diarrhea, constipation, nausea, vomiting, oral and small intestinal mucositis, colitis, proctitis, loss of appetite., etc. These gastrointestinal side effects can reduce patients' chances of recovery by impairing their ability to obtain adequate nutrition, which is essential for optimizing their capacity to fight the disease. Due to poor patient tolerance of these complications, chemotherapy-induced gastrointestinal side effects often lead to dose reduction, treatment interruption, or regimen modification-ultimately compromising patient outcomes and overall survival.

For example, the incidence of diarrhea induced by conventional chemotherapy agents such as fluorouracil and irinotecan has been reported to be as high as 50-80%. Stein, Ther. Adv. Med. Oncol. 2010, 2, 51-63; McQuade et al., Front Pharmacol. 2016, 7, 414. According to literature reports, chemotherapy-induced diarrhea (CID) leads to treatment regimen modifications in approximately 60% of colorectal cancer patients, including dose reductions, treatment delays, and therapy discontinuation. Arbuckle et al., Oncologist 2000, 5, 250-9; Dranitsaris et al., Can. J. Gastroenterol. 2005, 19, 83-7 .

Current clinical guidelines recommend loperamide as the first-line treatment for CID symptom control, though its efficacy remains limited for severe cases. For grade 3-4 diarrhea, octreotide is the consensus-recommended therapy. Other treatments, such as budesonide, probiotics, or antibiotics have not been shown to be effective in preventing or alleviating CID.

The incidence of chemotherapy-induced constipation (CIC) is difficult to estimate, CIC is reported to affect approximately 16% of cancer patients, and increasing evidence suggests that CIC significantly affects patients' quality of life. Current therapeutic interventions for managing chemotherapy-induced constipation (CIC) include oral and/or rectal administration of bulk-forming, stool-softening, osmotic, stimulant, and lubricant laxatives.

Overall, current treatments for CIC and CID are aimed at reducing the severity of symptoms rather than combating the dysfunctional pathogenesis (Andreyev et al., Lancet Oncol. 2014, 15, e447-e60; McQuade et al. , Front Pharmacol , 2016 , 7, 414) . A more effective approach to reducing gastrointestinal side effects in these patients involves protecting the gastrointestinal epithelium from primary damage and decreasing normal cell susceptibility to chemotherapeutic agents.

Therefore, there is an urgent need to develop effective drugs that can effectively treat, prevent or alleviate chemotherapy-induced gastrointestinal side effects, such as CIC and CID, while reducing damage to the gastrointestinal tract or other parts of the body; Moreover, the compound exhibits localized activity at the target site without significant systemic effects. It is suitable for oral administration and achieves therapeutic exposure in the gastrointestinal tract with minimal systemic exposure

### SUMMARY OF THE DISCLOSURE

The present application provides a CDK inhibitor. The CDK inhibitor is a CDK4/6 inhibitor. The CDK inhibitor is a compound shown in formula (I), which may be a pharmaceutically available salt, bioactive metabolite, solvate, hydrate, prodrug, racemate, enantiomer or stereoisomer. In the present application, the compound of formula (I) has properties that distinguish it from other CDK4/6 inhibitors in that the concentration of the compound in tissues in the gastrointestinal tract is much higher than the concentration of CDK4/6 inhibitors in the blood. This pharmacological property enables therapeutic efficacy in the gastrointestinal tract while significantly reducing CDK4/6 inhibitors' physiological effects on systemic organs, thereby minimizing associated adverse effects. The ultimate outcome is gastrointestinal-specific treatment without extra systemic physiological impact, substantially improving medication safety.

In one aspect, the present application provides a compound of formula (I), a pharmaceutically acceptable salt, bioactive metabolite, solvate, hydrate, prodrug, racemate, enantiomer or stereoisomer thereof, 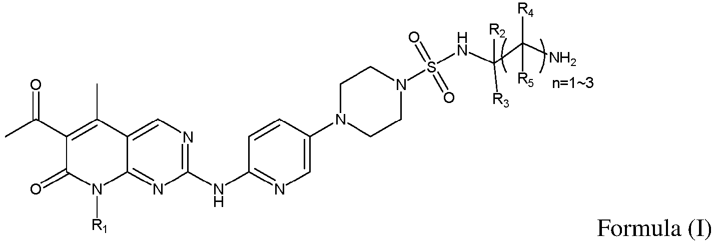 wherein,
n is 1, 2 or 3,
R₁ is selected from: hydrogen, substituted or unsubstituted alkyl, substituted or unsubstituted cycloalkyl, substituted or unsubstituted alkenyl;
R₂, R₃, R₄, and R₅ are each independently selected from: hydrogen, substituted or unsubstituted alkyl, substituted or unsubstituted cycloalkyl, and substituted or unsubstituted alkenyl;
Or any two groups of R₂, R₃, R₄ and R₅ are joined to form a ring.

In certain embodiments, the substitution comprises heteroatom substitution.

In certain embodiments, the substitution includes alkyl, cycloalkyl, alkenyl substitution.

In certain embodiments, R₂, R₃, R₄, and R₅ are each independently selected from the group consisting of C₁₋₁₀ substituted or unsubstituted alkyl, C₁₋₁₀ substituted or unsubstituted cycloalkyl, and C₁₋₁₀ substituted or unsubstituted alkenyl.

In certain embodiments, R₂, R₃, R₄, and R₅ are each independently selected from C₁₋₅ substituted or unsubstituted alkyl groups.

In certain embodiments, each R₄ is the same or different.

In certain embodiments, each R₅ is the same or different.

In certain embodiments, any two of the groups R₂, R₃, R₄ and R₅ are joined to form an alkane ring.

In certain embodiments, the compound of formula (I) comprises a structure selected from the group consisting of:

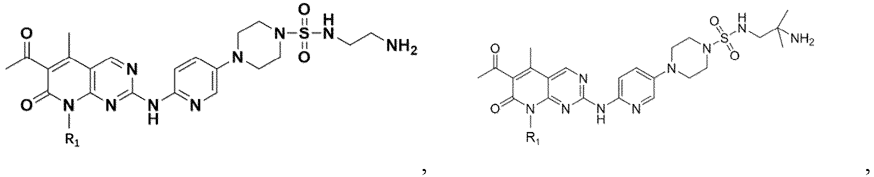

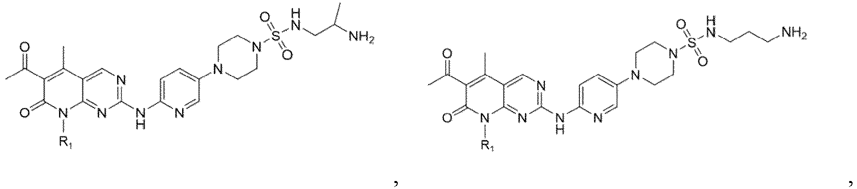

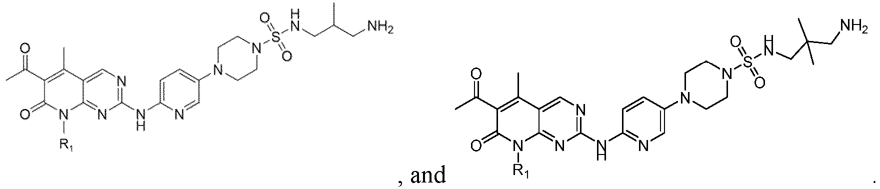

In certain embodiments, the R₁ is selected from: C₁₋₁₀ substituted or unsubstituted alkyl, C₁₋₁₀ substituted or unsubstituted cycloalkyl, C₁₋₁₀ substituted or unsubstituted alkenyl.

In certain embodiments, the R₁ is a substituted or unsubstituted cycloalkyl.

In certain embodiments, the R₁ is cyclopentyl.

In certain embodiments, n is 1.

In certain embodiments, n is 2.

In certain embodiments, n is 3.

In certain embodiments, the compound of formula (I) comprises a structure selected from the group consisting of:

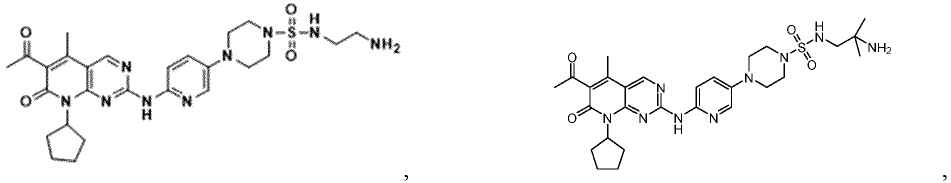

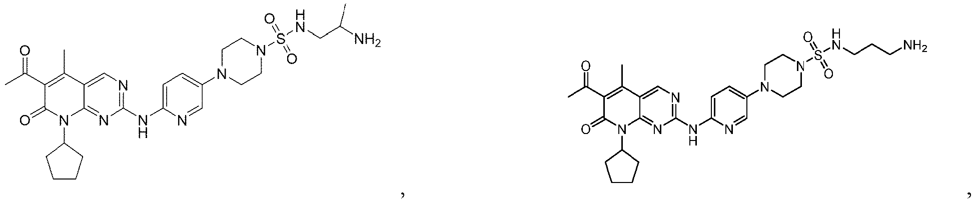

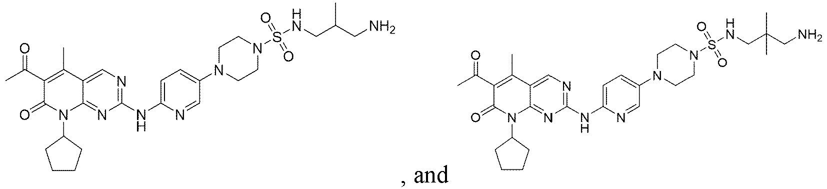

In certain embodiments, the compound, or a pharmaceutically acceptable salt, bioactive metabolite, solvate, hydrate, prodrug, racemate, enantiomer or stereoisomer thereof, which is a cyclin-dependent kinase (CDK) inhibitor.

In certain embodiments, the CDK inhibitor is a CDK4 /6 inhibitor.

In another aspect, the present application provides a method for preparing the compound, or a pharmaceutically acceptable salt, bioactive metabolite, solvate, hydrate, prodrug, racemate, enantiomer or stereoisomer thereof.

In another aspect, the present application provides a composition comprising the compound, a pharmaceutically acceptable salt, bioactive metabolite, solvate, hydrate, prodrug, racemate, enantiomer or stereoisomer thereof.

In certain embodiments, the composition further comprises an optional pharmaceutically acceptable carrier.

In certain embodiments, the composition further comprises one or more additional active ingredients.

In certain embodiments, the composition is formulated as an oral dosage form.

On the other hand, the present application also provides the compound, or a pharmaceutically acceptable salt, bioactive metabolite, solvate, hydrate, prodrug, racemate, enantiomer or stereoisomer thereof, or the composition in the manufacture of a medicament for preventing, alleviating and/or treating a disease and/or disorder.

In certain embodiments, wherein the disease and/or disorder comprises chemotherapy-associated gastrointestinal side effects.

In certain embodiments, wherein the chemotherapy comprises administration of a chemotherapeutic agent.

In certain embodiments, wherein the chemotherapeutic agent is a cytotoxic agent.

In certain embodiments, wherein the chemotherapeutic agent is selected from one or more of the following groups: DNA synthesis inhibitors, RNA synthesis inhibitors, protein synthesis inhibitors, cell division inhibitors, DNA base analogs, topoisomerase inhibitors and/or telomerase synthesis inhibitors.

In certain embodiments, wherein the chemotherapeutic agent is selected from fluorouracil, oxaliplatin, topotecan, irinotecan, tetrahydrofolate, docetaxel, gemcitabine, carboplatin, cisplatin, etoposide, methotrexate, doxorubicin, cytarabine, vinorelbine and capecitabine, or any combination thereof.

In certain embodiments, wherein the chemotherapeutic agent is administered continuously and/or non-continuously.

In certain embodiments, wherein the chemotherapy is used in combination with one or more other therapies.

In certain embodiments, wherein the chemotherapy-associated gastrointestinal side effects comprise gastrointestinal side effects caused by chemotherapy.

In certain embodiments, wherein the chemotherapy-associated gastrointestinal side effects comprise gastrointestinal adverse events that occur or worsen after administration of the chemotherapeutic agent.

In certain embodiments, wherein, in the absence of preventive or therapeutic intervention, the gastrointestinal adverse events occur or worsen at about 1 hour, about 2 hours, about 3 hours, about 4 hours, about 5 hours, about 6 hours, about 7 hours, about 8 hours, about 9 hours, about 10 hours, about 11 hours, about 12 hours, about 1 day, about 2 days, about 4 days, about 7 days, about 2 weeks, about 3 weeks, about 1 month, about 2 months, or longer after administration of the chemotherapeutic agent.

In certain embodiments, wherein the gastrointestinal side effects comprise gastric mucosal injury diseases and/or intestinal mucosal injury diseases.

In certain embodiments, wherein the gastrointestinal side effects comprise diarrhea, abdominal pain, nausea, vomiting, mucositis, loss of appetite, gastric ulcer, gastritis, constipation, enteritis, intestinal perforation, intestinal hemorrhage, ulcer and/or intestinal necrosis.

In certain embodiments, wherein the gastrointestinal side effects comprise diarrhea and/or constipation.

In certain embodiments, wherein the severity of the gastrointestinal side effects is grade 1 or higher, grade 2 or higher, grade 3 or higher, grade 4 or higher, or grade 5 in accordance with NCI-CTCAE criteria.

In certain embodiments, wherein the medicament substantially does not affect the therapeutic efficacy of the chemotherapy.

In certain embodiments, wherein the medicament is administered about 0.5 hours, about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours or more prior to the administration of the chemotherapy.

In certain embodiments, wherein the medicament is administered about 0.5 hours, about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours or more after the chemotherapy.

In certain embodiments, wherein the medicament is administered concurrently with the chemotherapy.

In certain embodiments, wherein the medicament is administered single or multiple doses.

In certain embodiments, wherein the medicament is formulated for oral administration, intravenous injection, subcutaneous injection, intraperitoneal injection and/or intramuscular injection.

In certain embodiments, wherein the medicament is formulated as a tablet and/or a capsule.

On the other hand, the present application provides a method for preventing, alleviating and/or treating a disease and/or disorder in a subject, comprising administering to a subject in need thereof the compound, or a pharmaceutically acceptable salt, bioactive metabolite, solvate, hydrate, prodrug, racemate, enantiomer or stereoisomer thereof, or the composition.

In certain embodiments, wherein the disease and/or disorder comprises chemotherapy-associated gastrointestinal side effects.

In certain embodiments, wherein the chemotherapy comprises administration of a chemotherapeutic agent.

In certain embodiments, wherein the chemotherapeutic agent is a cytotoxic agent.

In certain embodiments, wherein the chemotherapeutic agent is selected from one or more of the following groups: DNA synthesis inhibitors, RNA synthesis inhibitors, protein synthesis inhibitors, cell division inhibitors, DNA base analogs, topoisomerase inhibitors and/or telomerase synthesis inhibitors.

In certain embodiments, wherein the chemotherapeutic agent is selected from fluorouracil, oxaliplatin, topotecan, irinotecan, tetrahydrofolate, docetaxel, gemcitabine, carboplatin, cisplatin, etoposide, methotrexate, doxorubicin, cytarabine, vinorelbine and capecitabine, or any combination thereof.

In certain embodiments, wherein the chemotherapeutic agent is administered continuously and/or non-continuously.

In certain embodiments, wherein the chemotherapy is used in combination with one or more other therapies.

In certain embodiments, wherein the chemotherapy-associated gastrointestinal side effects comprise gastrointestinal side effects caused by chemotherapy.

In certain embodiments, wherein the chemotherapy-associated gastrointestinal side effects comprise gastrointestinal adverse events that occur or worsen after the administration of a chemotherapeutic agent.

In certain embodiments, wherein, in the absence of preventive or therapeutic intervention, the gastrointestinal adverse events occur or worsen at about 1 hour, about 2 hours, about 3 hours, about 4 hours, about 5 hours, about 6 hours, about 7 hours, about 8 hours, about 9 hours, about 10 hours, about 11 hours, about 12 hours, about 1 day, about 2 days, about 4 days, about 7 days, about 2 weeks, about 3 weeks, about 1 month, about 2 months, or longer after administration of the chemotherapeutic agent.

In certain embodiments, wherein the gastrointestinal side effects comprise gastric mucosal injury diseases and/or intestinal mucosal injury diseases.

In certain embodiments, wherein the gastrointestinal side effects comprise diarrhea, abdominal pain, nausea, vomiting, mucositis, loss of appetite, gastric ulcer, gastritis, constipation, enteritis, intestinal perforation, intestinal hemorrhage, ulceration and/or intestinal necrosis.

In certain embodiments, wherein the gastrointestinal side effects comprise diarrhea and/or constipation.

In certain embodiments, wherein the severity of the gastrointestinal side effects is grade 1 or higher, grade 2 or higher, grade 3 or higher, grade 4 or higher, or grade 5 in accordance with NCI-CTCAE criteria.

In certain embodiments, the medicament substantially does not affect the therapeutic efficacy of the chemotherapy.

In certain embodiments, the medicament is administered about 0.5 hours, about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours or more prior to the administration of the chemotherapy.

In certain embodiments, the medicament is administered about 0.5 hours, about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours or more after administration of the chemotherapy.

In certain embodiments, the medicament is administered concurrently with the chemotherapy.

In certain embodiments, the medicament is administered single or multiple doses.

In certain embodiments, the compound, or a pharmaceutically acceptable salt, bioactive metabolite, solvate, hydrate, prodrug, racemate, enantiomer or stereoisomer thereof, or the composition is administered at a dose of about 10-1000 mg /kg.

In certain embodiments, wherein the subject is a cancer patient.

In another aspect, the present application provides a kit comprising a compound described herein, or a pharmaceutically acceptable salt, bioactive metabolite, solvate, hydrate, prodrug, racemate, enantiomer or stereoisomer thereof.

In certain embodiments, the kit further comprises instructions for use of the compound, or a pharmaceutically acceptable salt, bioactive metabolite, solvate, hydrate, prodrug, racemate, enantiomer or stereoisomer thereof.

In certain embodiments, the kit further comprises one or more pharmaceutically acceptable carriers.

Those skilled in the art will be able to readily gain insight into other aspects and advantages of the present application from the detailed description below. Only exemplary embodiments of the present application are shown and described in the detailed description below. As will be recognized by those skilled in the art, the contents of this application enable those skilled in the art to make changes to the specific embodiments disclosed without departing from the spirit and scope of the invention to which this application relates. Accordingly, the descriptions in the specification of this application are merely exemplary and are not intended to be limiting.

### DETAILED DESCRIPTION

The following illustrates the embodiments of the presently applied invention by particular specific embodiments, and other advantages and effects of the presently applied invention may be readily appreciated by those familiar with the art from what is disclosed in this specification.

### Term definition

In the present application, the term " prodrug " is also referred to as " precursor drug", which generally refers to an agent or compound that is converted into a parent drug or active drug molecule in vivo. For example, the "prodrug" can be a precursor of another pharmaceutically active molecule, which, after being administered to a subject, produces the other pharmaceutically active molecule in vivo through chemical or physiological processes such as solvolysis or enzymatic cleavage, or under physiological conditions.

As used herein, the term "substituted or unsubstituted alkyl " includes straight or branched alkyl groups that do not contain any substituents, and also includes straight or branched alkyl groups that contain one or more non-hydrogen substituents. For example, the alkyl group generally includes a saturated aliphatic hydrocarbon group with a specified number of carbon atoms, which may be branched or straight chain. The term " alkyl " also refers to a non-aromatic cycloalkyl group. For example, the alkyl group may have 1-20 carbons (i.e., C₁ -C₂₀). For example, C₁-C₁₀ as in " C₁-C₁₀ alkyl " is defined as a group including 1, 2, 3, 4, 5, 6, 7, 8, 9 or 10 carbons that are straight, branched or cyclically arranged (i.e., cycloalkyl). The term " cycloalkyl " refers to a monocyclic saturated aliphatic hydrocarbon group with a specified number of carbon atoms. For example, " alkyl " specifically includes methyl, ethyl, n-propyl, isopropyl, n-butyl, tert-butyl, isobutyl, pentyl, hexyl, heptyl, octyl, nonyl, decyl and the like, as well as cycloalkyl including cyclopropyl, methyl-cyclopropyl, 2,2-dimethyl-cyclobutyl, 2-ethyl-cyclopentyl, cyclohexyl and the like.

In the present application, the term "bioactive metabolite" generally refers to a compound (e.g., a compound of the present application) that is processed in vivo by being metabolized in the human or animal body or cell to produce a metabolite. For example, the "bioactive metabolite" may include derivatives of any structural formula produced in an individual after administration of the parent compound. These derivatives may be produced from the parent compound by various biochemical transformations in the individual, such as oxidation, reduction, hydrolysis, or combination, and include, for example, oxides and demethyl derivatives. Metabolites of the compounds of the present invention may be identified using conventional techniques known in the prior art. See, e.g., Bertolini, G. et al., J. Med. Chem. 40:2011-2016 (1997); Shan, D. et al., J. Pharm. Sci. 86(7):765-767; Bagshawe K., Drug Dev. Res. 34:220-230 ( 1995); Bodor, N., Advances in Drug Res. 13:224-331 (1984); Bundgaard, H., Design of Prodrugs (Elsevier Press 1985); and Larsen, IK, Design and Application of Prodrugs, Drug Design and Development (Krogsgaard-Larsen et al., eds., Harwood Academic Publishers, 1991). It should be understood that chemical compounds which are metabolites of compounds of formula (I) or their tautomers, prodrugs and stereoisomers, as well as pharmaceutically acceptable salts, esters and prodrugs of any of them are included in the present application.

In this application, the term " pharmaceutically acceptable salt " generally refers to a salt that retains the biological effectiveness and properties of a free base or free acid and is biologically or otherwise desirable. The salt may be a salt formed with an inorganic acid or a salt formed with an organic acid. In addition, the pharmaceutically acceptable salt may be prepared by adding an inorganic base or an organic base to a free acid.

In the present application, the term "CDK inhibitor" generally refers to any molecules that have been known in the art or will be found in the future and are capable of blocking, reducing or inhibiting the activity or function of CDK (cyclin-dependent kinase), including, but not limited to, a small molecular compound, a polynucleotide (e.g., DNA or RNA), and/or a polypeptide (e.g., an antibody or an antigen-binding portion thereof). The CDK inhibitor can act directly on CDK, e.g. through binding to CDK, or act indirectly, e.g. through interfering with the interaction between CDK and its ligand, inhibiting cyclin or inhibiting the activity of the substrate. CDK is a class of kinase in the protein kinase family, which can be used to regulate the cell cycle, and are also involved in the regulation of transcription, mRNA processing and the differentiation of nerve cells. The CDK in the present application may refer to an intact CDK or a kinase domain fragment, and also encompasses CDKs from various vertebrates (e.g., human, monkey, mouse, dog, rabbit, etc.). According to different bound cyclins, CDK can be divided into different types. For example, there are four CDKs which are evidently involved in cell proliferation: CDK1, which mainly regulates the transition from phase G2 to phase M; CDK2, CDK4 and CDK6, which regulate the transition from phase G1 to phase S.

In the present application, the term "CDK4/6 inhibitor" generally refers to an inhibitor that acts on both CDK4 and CDK6 in the CDK family. In all the members of CDK family, it mainly has inhibition effects against CDK4 and CDK6, but not excluding the possibility of having inhibition effects on members of the CDK family other than CDK4 and CDK6, and also not excluding the possibility of having inhibition effects on other molecules besides the CDK family. It is not required that the CDK4/6 inhibitor has the same or similar effects against CDK4 and CDK6.

In the present application, the term "chemotherapy" generally refers to a therapy that uses a chemotherapeutic agent to treat tumors, which can lead to the death of cancer cells, or interfere with the division, repair, growth and/or function of cancer cells. The medicament used in the chemotherapy is a chemotherapeutic agent. The chemotherapeutic agent comprises a chemical or biological substance which can cause the death of cancer cells or interfere with the growth, division, repair and/or function of cancer cells. For example, the chemotherapy may comprise cytotoxic agents, cell inhibitors and anti-tumor agents which can kill tumor cells, inhibit the growth or metastasis of tumor cells or disrupt the cell cycle of rapidly proliferating cells. The chemotherapeutic agent comprises natural compounds found in animals and plants, or artificial chemical substances. In the present application, the chemotherapy or chemotherapeutic agents have low selectivity on tumor cells and normal cells, and therefore can also interfere with the growth, division, repair and/or function of normal cells (e.g., bone marrow cells, hair follicle cells or digestive tract cells), finally resulting in the death of normal cells.

In the present application, the term " pharmaceutically acceptable " generally refers to compounds, materials, compositions and/or dosage forms that are suitable for contact with human and animal tissues without excessive toxicity, irritation, allergic response or other problems or complications within the scope of reasonable medical judgment, and have a reasonable benefit/risk ratio. In some embodiments, pharmaceutically acceptable compounds, materials, compositions and/or dosage forms refer to those approved by regulatory agencies (such as the U.S. Food and Drug Administration, the National Medical Products Administration of China or the European Medicines Agency) , or are listed in an officially recognized pharmacopeia (such as the United States Pharmacopeia, the Chinese Pharmacopoeia, or the European Pharmacopoeia) for use in animals (more specifically, in humans).

In the present application, examples of the chemotherapeutic agents may include, but not limited to, alkylating agents, e.g., mechlorethamine, ethyleneimine compounds, alkyl sulfonates and other compounds having alkylating effects, e.g. nitrosourea, cisplatin and dacarbazine; antimetabolites, e.g., folic acid, purine or pyrimidine antagonists; mitotic inhibitors, e.g. vinca alkaloid and podophyllotoxin derivatives; cytotoxic antibiotics and camptothecin derivatives. The chemotherapeutic agents may also include amifostine cisplatin, dacarbazine (DTIC), dactinomycin, streptomycin, cyclophosphamide, carmustine (BCNU), lomustine (CCNU), doxorubicin, doxorubicin liposome, gemcitabine, erythromycin, daunorubicin liposome procarbazine, mitomycin, cytarabine, etoposide, methotrexate, 5-fluorouracil (5-FU), vinblastine, vincristine, bleomycin, paclitaxel, Docetaxel, Aldesleukin, Asparaginase, busulfan, carboplatin, cladribine, camptothecin, CPT-11, 10-hydroxyl-7-ethyl-camptothecin (SN38), floxuridine, fludarabine, hydroxyurea, ifosfamide, idarubicin, Mesna, interferon α, interferon β, irinotecan, mitoxantrone, topotecan, Lupron, megestrol, Melphalan, mercaptopurine, plicamycin, mitotane, Oncaspar, pegaspargase, pentostatin, pipobroman, plicamycin, streptozocin, tamoxifen, teniposide, testosterone, thioguanine, thiotepa, uramustine, vinorelbine, chlorambucil aromatase inhibitor and combinations thereof.

In the present application, the term "cytotoxic agent" generally refers to a reagent for inhibiting the biological process of cells or reducing the viability or proliferative potential of cells. The cytotoxic agent may act in various ways, for example, but not limited to, through inducing DNA damages, inducing cell cycle arrest, inhibiting DNA synthesis, inhibiting transcription, inhibiting translation or protein synthesis, inhibiting cell division or inducing apoptosis.

In the present application, the term "does not substantially affect" generally refers to that as compared to the therapeutic effect of using the chemotherapy alone, the use of the combination of the medicament and the chemotherapy of the present application has a comparable therapeutic effect, or does not produce a significant disadvantage. For example, for any subject, as compared to the therapeutic effect of using the chemotherapy alone, the use of the combination of the medicament and the chemotherapy causes the same degree of tumor size reduction, or the reduction degree is not less than about 5%, not less than about 4%, not less than about 3%, not less than about 2%, not less than about 1%, not less than about 0.5%, not less than about 0.1%, not less than about 0.01%, not less than about 0.001% or smaller.

In the present application, the term "continuous administration" generally refers to repeated administration of the same medicament every day. Repeated administration every day may be once a day, twice a day, three times or more times a day. For example, continuous administration may be administration once a day for at least more than 2 days. For example, a continuous administration time of 2 days may mean administration once a day (twice, three times or more) continuously for 2 days. For example, a continuous administration time of 3 days may mean administration once a day (twice, three times or more) continuously for 3 days. For example, a continuous administration time of 5 days may mean administration once a day (twice, three times, or more) continuously for 5 days. For example, a continuous administration time of 7 days may mean administration once a day (twice, three times, or more) continuously for 7 days. For example, a continuous administration time of 10 days may mean administration once a day (twice, three times, or more) continuously for 10 days. For example, a continuous administration time of 14 days may mean administration once a day (twice, three times, or more) continuously for 14 days. For example, a continuous administration time of more than 7 days may mean administration once a day (twice, three times, or more) continuously for more than 7 days.

In the present application, the term "noncontinuous administration" generally refers to an administration mode in which the same medicament is not administered every day in an administration cycle, also known as intermittent administration and/or pulsed administration. Noncontinuous administration may be regular or irregular. In noncontinuous administration, in the case that the administration cycle is longer than 7 days, for any continuous period of time, if the administration is performed every day and continued for 7 days or above, it also belongs to the case of "a continuous administration time of 7 days or above".

In the present application, the term "gastrointestinal adverse events" generally refers to harmful and undesirable reactions, effects, actions, consequences, results or influences associated with the gastrointestinal tract or displayed in the gastrointestinal tract area, which are caused by a certain medicament or other medical treatments such as chemotherapy or surgery. They are also known as gastrointestinal adverse effects, gastrointestinal adverse influences or gastrointestinal adverse consequences. The gastrointestinal adverse events comprise adverse events in various parts (e.g., digestive tracts and glands) of the digestive system. For example, gastrointestinal adverse events may include, but not limited to, abdominal distension, abdominal pain, anal fissure, anal fistula, anal hemorrhage, anal mucositis, anal necrosis, anal pain, anal stenosis, anal ulcer, ascites, belching, cecal hemorrhage, cheilitis, chylous ascites, colitis, colonic fistula, colonorrhagia, colonic obstruction, colonic perforation, colonic stenosis, colonic ulcer, constipation, dental caries, diarrhea, dry mouth, duodenal fistula, duodenal hemorrhage, duodenal obstruction, duodenal perforation, duodenal stenosis, duodenal ulcer, dyspepsia, dysphagia, enterocolitis, intestinal fistula, esophageal fistula, esophageal hemorrhage, esophageal necrosis, esophageal obstruction, esophageal pain, esophageal perforation, esophageal stenosis, esophageal ulcer, esophageal variceal hemorrhage, oesophagitis, fecal incontinence, flatulence, gastric fistula, gastrorrhagia, gastric necrosis, gastric perforation, gastric stenosis, gastric ulcer, gastritis, gastroesophageal reflux disease, gastrointestinal diseases (others, please noted), gastrointestinal fistula, gastrointestinal pain, gastroparesis, gum pain, hemorrhoidal hemorrhage, haemorrhoids, ileal fistula, ileal hemorrhage, ileocleisis, ileal perforation, ileal stenosis, ileal ulcer, ileus, intraperitoneal hemorrhage, jejunal fistula, jejunal hemorrhage, jejunal obstruction, jejunal perforation, jejunal stenosis, jejunal ulcer, chilalgia, lower gastrointestinal hemorrhage, malabsorption, oral mucositis, nausea, gastric obstruction, oral fistula, oral dysaesthesia, oral hemorrhage, oral pain, pancreatic-duct stenosis, pancreatic fistula, pancreatic hemorrhage, pancreatic necrosis, pancreatitis, periodontal diseases, peritoneal necrosis, rectitis, rectal rupture, rectofistula, rectal hemorrhage, rectal mucositis, rectal necrosis, rectal obstruction, rectal pain, rectal perforation, rectostenosis, rectal ulcer, retroperitoneal hemorrhage, salivary duct inflammation, salivary gland fistula, small intestinal mucositis, small intestinal obstruction, small intestinal perforation, small intestinal stenosis, small intestinal ulcer, abdominal pain, dental developmental disturbance, tooth discoloration, toothache, appendicitis, upper gastrointestinal hemorrhage, visceral artery ischemia, vomiting.

In the present application, the term "gastrointestinal side effect" generally refers to harmful and adverse effects related to or manifested in the gastrointestinal tract caused by prophylactic or therapeutic agents. Adverse effects are always undesirable, but undesirable effects are not necessarily adverse. The adverse effects of prophylactic or therapeutic agents may be harmful, uncomfortable, or hazardous. Gastrointestinal side effects include adverse effects involving various parts of the digestive system (e.g., digestive tract and gland). Chemotherapy-associated gastrointestinal side effects may refer to any abnormal clinical manifestations of gastrointestinal tract associated with the time of using the chemotherapeutic agent, but there may be no causal relationship between these abnormal manifestations and the administration of the chemotherapeutic agent.

In the present application, the term "gastric mucosal injury diseases" generally refers to the diseases or disorders with symptoms of gastric mucosal injury, which can include abnormal color, hemorrhage spots, congestion and erosion of the gastric mucosa.

In the present application, the term "intestinal mucosal injury diseases" generally refers to the diseases or disorders with symptoms of intestinal mucosal injury, which can include abnormal color, hemorrhage spots, congestion and erosion of the intestinal mucosa.

In the present application, the term "diarrhea" generally refers to a disease or disorder with the features of increased peristalsis frequency and/or relaxation or watery peristalsis. In the present application, the diarrhea may occur or deteriorate after administration of the chemotherapeutic agent. In some cases, diarrhea can also be evaluated referring to animal experiments: for example, the severity of diarrhea is scored in accordance with the method of Akinobu Kurita (Cancer Chemother Pharmacol 2000; 46:211-20.), wherein, Grade 0: normal stool; Grade 1: mild diarrhea, slightly soft stool and wet stool; Grade 2: moderate diarrhea, loose stool with slight perianal staining; Grade 3: severe diarrhea, loose watery stool with severe perianal staining.

In the present application, the term "constipation" generally refers to a disease or disorder with the features of irregular defecation, infrequent defecation or difficult defecation of intestines. In the present application, the constipation may occur or deteriorate after administration of the chemotherapy.

In the present application, the term "in the absence of prevention or treatment" generally refers to a condition without performing prevention or treatment on gastrointestinal adverse events, and without taking measures to make the gastrointestinal adverse events to be weakened or disappear. The measures to make the gastrointestinal adverse events to be weakened or disappear may be, for example, administration of a medicament or other means to prevent or treat gastrointestinal adverse events, stopping the administration of medicaments or other means that cause gastrointestinal adverse events, including administration of the CDK inhibitor or the medicament of the present application. In the present application, after administering the chemotherapy of the present application, if no measures are taken to make the gastrointestinal adverse events to be weakened or disappear, the gastrointestinal adverse events will occur or deteriorate 1 hour, 2 hours, 3 hours, 4 hours, 5 hours, 6 hours, 7 hours, 8 hours, 9 hours, 10 hours, 11 hours, 12 hours, 1 day, 2 days, 4 days, 7 days, 2 weeks, 3 weeks, 1 month, 2 months or longer after the administration of the chemotherapy. If the CDK inhibitor or the medicament of the present application is administered, the gastrointestinal adverse events may be weakened or disappear.

In the present application, the term "NCI-CTCAE" generally refers to a standardized definition of adverse events issued by National Cancer Institute (NCI)-Common Terminology Criteria for Adverse Events (CTCAE), which is used to describe the severity of organ toxicity in patients treated for cancer. With the development of scientific evidence, the criteria can be updated continuously. In the present application, diarrhea can be evaluated with reference to the evaluation criteria of "NCI-CTCAE" in some cases. NCI-CTCAE may comprise any version of "NCI-CTCAE". In CTCAE V5.0, constipation and diarrhea are defined into 5 grades respectively, as shown in the table below.

### Standardized definition on the grading of constipation in CTCAE V5.0

| CTCAE Grading | Criteria |
|---|---|
| Grade 1 | Incidental or intermittent symptoms; occasionally employing stool softener, laxatives, antisecosis or enteroclvsis |
| Grade 2 | Persistent symptoms in need of long-term use of laxatives or enteroclysis; limited instrumental activities of daily living (ADL) |
| Grade 3 | Constipation with manual evacuation indications; limited self-care |
| Grade 4 | Life-threatening consequences; indicating emergency interventions |
| Grade 5 | Death |

### Standardized definition on the grading of diarrhea in CTCAE V5.0

| CTCAE Grading | Criteria |
|---|---|
| Grade 1 | An increasement of < 4 times of defecation compared with the basic standard every day; compared with the baseline, the output of neostomy increases slightly |
| Grade 2 | An increasement of 4-6 times of defecation compared with the basic standard every day; compared with the baseline, the output of neostomy increases moderately; limited instrumental ADL |
| Grade 3 | An increasement of >= 7 times of defecation compared with the basic standard every day; indicating hospitalization; compared with the baseline, the output of neostomy increases significantly; limited self-care |
| Grade 4 | Life-threatening consequences; indicating emergency interventions |
| Grade 5 | Death |

In the present application, the term "cancer" generally refers to any medical conditions mediated by the growth, proliferation or metastasis of tumors or malignant cells and causing solid tumors and nonsolid tumors (e.g., leukemia). The cancer in the present application may include, but not limited to, malignant epithelial tumors (epithelium-derived cancers), lung cancer (e.g., non-small cell lung cancer), breast cancer, skin cancer, bladder cancer, colon cancer, gastrointestinal (GI) cancer, prostatic cancer, pancreatic cancer, uterine cancer, cervical cancer, ovarian cancer, esophagus cancer, head and neck cancer, gastric cancer and throat cancer.

### Detailed description of the invention

### Compounds

In one aspect, the present application provides a compound of formula (I), and also provides a pharmaceutically acceptable salt, bioactive metabolite, solvate, hydrate, prodrug, racemate, enantiomer or stereoisomer thereof, 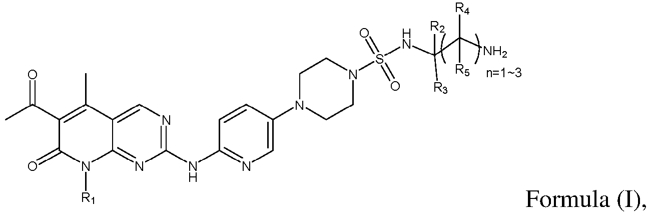 wherein,
n is 1 , 2 or 3,
R₁ is selected from: hydrogen, substituted or unsubstituted alkyl, substituted or unsubstituted cycloalkyl, substituted or unsubstituted alkenyl;
R₂, R₃, R₄, and R₅ are each independently selected from: hydrogen, substituted or unsubstituted alkyl, substituted or unsubstituted cycloalkyl, and substituted or unsubstituted alkenyl;
Or any two of R₂, R₃, R₄ and R₅ are joined to form a ring.

In the present application, the compound is a CDK inhibitor. For example, the compound is a CDK4/6 inhibitor.

In the compounds of the present application, n is 1-3. In certain embodiments, n is 1. In certain embodiments, n is 2. In certain embodiments, n is 3.

In the compounds of the present application, wherein R₂, R₃, R₄, R₅ are each independently selected from hydrogen, substituted or unsubstituted alkyl, substituted or unsubstituted cycloalkyl, substituted or unsubstituted alkenyl.

In certain embodiments, the R₂ is hydrogen.

In certain embodiments, the R₃ is hydrogen.

In certain embodiments, the R₄ is hydrogen. In certain embodiments, the R₄ is unsubstituted alkyl. In certain embodiments, the R₄ is optionally substituted alkyl.

In certain embodiments, the R₅ is hydrogen. In certain embodiments, the R₅ is unsubstituted alkyl. In certain embodiments, the R₅ is optionally substituted alkyl.

In certain embodiments, R₂, R₃, R₄, R₅ are each independently selected from: C₁₋₁₀ substituted or unsubstituted alkyl, C₁₋₁₀ substituted or unsubstituted cycloalkyl, or C₁₋₁₀ substituted or unsubstituted alkenyl. When the R₁ is a substituted group, it may be substituted with any other group.

In certain embodiments, R₁ may be a cyclopentyl group.

In the present application, R₂, R₃, R₄, R₅ may independently be selected from: hydrogen, substituted or unsubstituted alkyl, substituted or unsubstituted cycloalkyl, substituted or unsubstituted alkenyl. For example, R₂, R₃, R₄, R₅ may be hydrogen. For example, R₂, R₃, R₄, R₅ may independently be unsubstituted alkyl. For example, R₂, R₃, R₄, R₅ may independently be substituted alkyl. For example, R₂, R₃, R₄, R₅ may independently be unsubstituted cycloalkyl. For example, the R₂, R₃, R₄, R₅ may independently be substituted cycloalkyl. For example, R₂, R₃, R₄, R₅ may independently be substituted alkenyl. For example, R₂, R₃, R₄, R₅ may independently be unsubstituted alkenyl. In the present application, when R₂, R₃, R₄, R₅ are substituted groups, it may be substituted with any other groups. For example, the substituent group may be an alkyl, cycloalkyl, alkenyl, or heteroatom group. For example, the heteroatom may be selected from N, S, O, and P. In the present application, R₁ may be a substituted group in which the substituents are directly bonded to a carbon chain or a heteroatom. For example, the number of substituents may be up to full substitution.

In certain embodiments, R₄ and R₅ may each independently be an unsubstituted alkyl. For example, R₄ and R₅ may each be an unsubstituted methyl.

In certain embodiments, each of the R₄ may be the same. In certain embodiments, each of the R₄ may be different.

In certain embodiments, each of the R₅ may be the same.

In certain embodiments, each of the R₅ may be different.

In certain embodiments, any two of the R₂, R₃, R₄ and R₅ groups may be joined to form a ring. For example, any two of R₂, R₃, R₄ and R₅ are joined to form an alkane ring.

In the present application, the compound may comprise a structure selected from the following group:

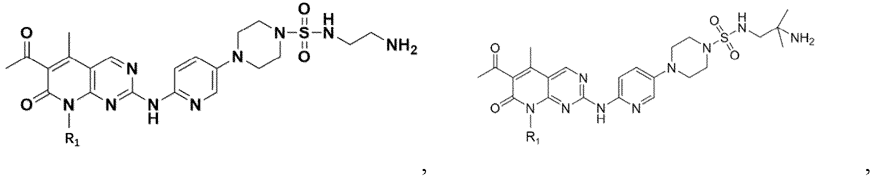

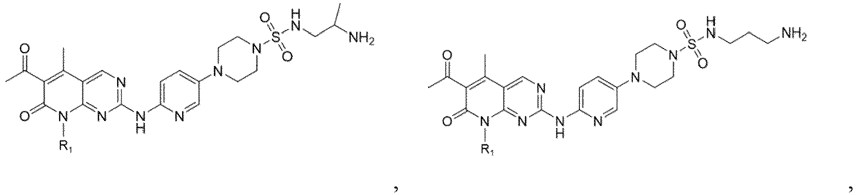

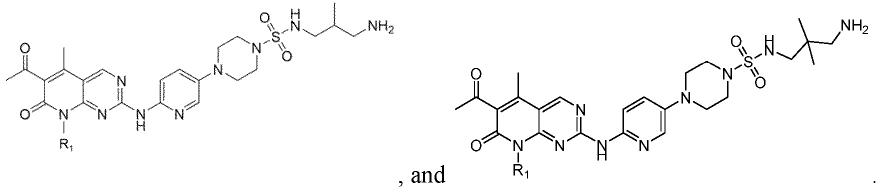

In the present application, the compound may comprise a structure selected from the following group:

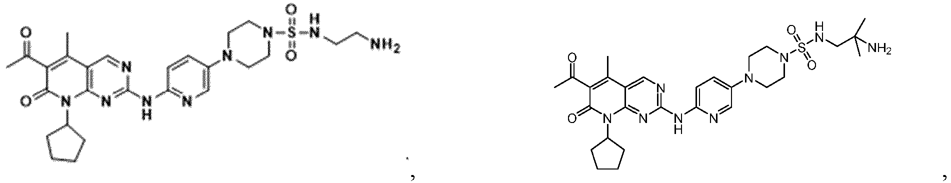

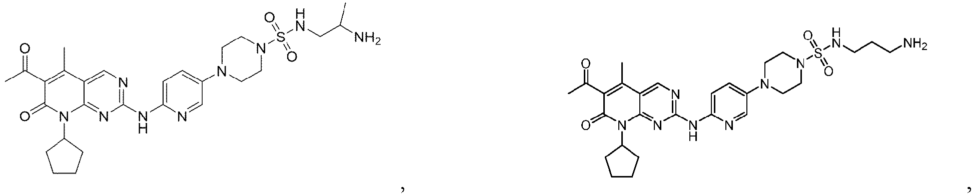

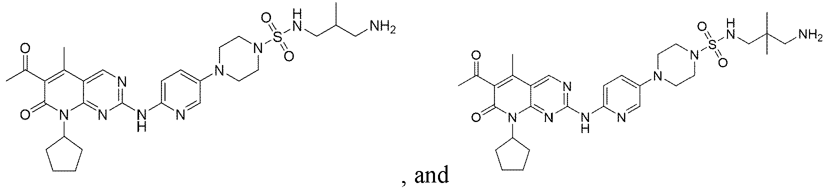

In the present application, when referring to the structure of the compound, it is intended to include isotopic variations at any position within the compound. For example, hydrogen atoms may include deuterium and tritium. For example, carbon atoms may include carbon-13. For example, nitrogen may include nitrogen-15.

In the present application, the compound may also include a pharmaceutically acceptable salt, bioactive metabolite, solvate, hydrate, prodrug, racemate, enantiomer or stereoisomer thereof.

### Chemotherapy

In the present application, the chemotherapeutic agent used in the chemotherapy may be any one kind of compound or medicament used for treating cancers selected from those known to persons skilled in the art. In terms of the mechanism of action, the chemotherapeutic agent may comprise a DNA synthesis inhibitor, an RNA synthesis inhibitor, a protein synthesis inhibitor, a cell division inhibitor, a DNA base analogue, a topoisomerase inhibitor and/or a telomerase synthesis inhibitor.

In the present application, the chemotherapeutic agent may be toxic to cells. In the present application, the chemotherapeutic agent may inhibit cell growth. In the present application, the administered chemotherapeutic agent may be a chemotherapeutic agent for DNA damages. In the present application, the chemotherapeutic agent is a protein synthesis inhibitor, a DNA-damage chemotherapeutic agent, an alkylating agent, a topoisomerase inhibitor, a RNA synthesis inhibitor, a DNA composite binding agent, a thiolate alkylating agent, a guanine alkylating agent, a tubulin binding agent, a DNA polymerase inhibitor, an anti-cancer enzyme, a RAC1 inhibitor, a thymidylic acid synthetase inhibitor, an oxazophosphorine compound, an integrin inhibitor, such as cilengitide, camptothecin or homocamptothecin, an antifolate, a folic acid antimetabolite, a telomerase inhibitor and/or a telomere DNA-binding compound.

For example, the alkylating agent may comprise alkyl sulfonates, such as busulfan, improsulfan and piposulfan; aziridines, such as benzodizepa, carboquone, meturedepa and uredepa; ethylene imines and methyl melamines, such as altretamine, triethylene melamine, triethylene phosphamide, triethylene thiophosphamide and trimethylol melamine; mechlorethamines, such as chlorambucil, chlornaphazine, cyclophosphamide, estramustine, dichloromethyl diethylamine, mechlorethamine oxide hydrochloride, Melphalan, novembichine, phenesterine, prednimustine, trofosfamide and uracil mustard; and nitrosoureas, such as carmustine, chlorozotocin, fotemustine, lomustine, nimustine and ranimustine. Other chemotherapeutic agents may comprise daunorubicin, doxorubicin, idarubicin, epirubicin, mitomycin and streptozotocin. Antimetabolites for the chemotherapy may comprise gemcitabine, mercaptopurine, thioguanine, cladribine, fludarabine phosphate, fluorouracil (5-FU), floxuridine, cytarabine, pentostatin, methotrexate, azathioprine, acyclovir, adenine β-1-D-arabinoside, amethopterin, aminopterin, 2-aminopurine, aphidicolin, 8-azaguanine, azaserine, 6-azauracil, 2'-azido-2'-deoxynucleoside, 5-bromodeoxycytidine, cytosine β-1-D-arabinoside, diazoxonorleucine, dideoxynucleoside, 5-fluorodeoxycytidine, 5-fluorodeoxyuridine and hydroxyurea.

For example, the protein synthesis inhibitor may comprise abrin, aurin tricarboxylic acid, chloramphenicol, colicin E3, cycloheximide, diphtheria toxin, edeine A, emetine, erythromycin, ethionine, fluoride, 5-fluorotryptophan, fusidic acid, guanylyl methylene bisphosphonate and guanylyl imino diphosphate, kanamycin, kasugamycin, kirromycin and O-methylthreonine. Other protein synthesis inhibitors comprise modeccin, neomycin, norvaline, pactamycin, paromomycine, puromycin, ricin, Shiga toxin, showdomycin, sparsomycin, spectinomycin, streptomycin, tetracycline, thiostreptone and trimethoprim.

For example, the DNA synthesis inhibitor may comprise an alkylating agent, such as dimethyl sulfate, mechlorethamine and sulfur mustard; an intercalating agent, such as acridine dyes, actinomycin, anthracenes, benzopyrene, ethidium bromide, propidium diiodide-intertwining; a topoisomerase inhibitor, such as irinotecan, teniposide, coumermycin, nalidixic acid, novobiocin and oxolinic acid; a cell division inhibitor, including demecolcine, mitoxantrone, colchicine, vinblastine and vincristine; and other medicaments, such as distamycin and netropsin.

In the present application, the chemotherapeutic agent may be a DNA composite binding agent, e.g., camptothecin or etoposide; a thiolate alkylating agent, e.g., nitrosourea, BCNU, CCNU, ACNU or fotesmustine; a guanine alkylating agent, e.g., temozolomide; a tubulin binding agent, such as vinblastine, vincristine, vinorelbine, vinflunine, cryptophycin 52, halichondrin such as halichondrin B, aplysiatoxin such as aplysiatoxin 10 and aplysiatoxin 15, hemiasterlins (such as hemiasterlin A and hemiasterlin B), colchicine, combrestatins, 2-methoxy estradiol, E7010, paclitaxel, Docetaxel, epothilone, discodermolide; a DNA polymerase inhibitor, e.g., cytarabine; an anti-cancer enzyme, e.g., asparaginase; a RAC1 inhibitor, e.g., 6-thioguanine; a thymidylic acid synthetase inhibitor, e.g., capecitabine or 5-FU; an oxazophosphorine compound, e.g., Endoxan; an integrin inhibitor, e.g., cilengitide; an antifolate, e.g., pralatrexate; a folic acid antimetabolite, e.g., pemetrexed; or camptothecin or homocamptothecin, e.g., diflomotecan.

In the present application, the CDK inhibitor can be used for treating the above one or more chemotherapy-associated gastrointestinal side effects. In the present application, the CDK inhibitor can be used for treating gastrointestinal side effects associated with the administration of the following chemotherapeutic agents: fluorouracil, oxaliplatin, irinotecan (CPT-11), docetaxel (DTX), gemcitabine (GEM), paclitaxel, carboplatin, doxorubicin (Dox), methotrexate (MTX), cytarabine (Ara-C), vinorelbine (NVB), topotecan (TP), etoposide and cisplatin, and any combination of the above.

In the present application, the CDK inhibitor can be used for treating gastrointestinal side effects associated with the administration of the following chemotherapeutic agents: fluorouracil, oxaliplatin, topotecan, irinotecan, tetrahydrofolic acid, docetaxel, gemcitabine, carboplatin, cisplatin, etoposide, methotrexate, doxorubicin, cytarabine, vinorelbine and capecitabine, and any combination of the above.

In the present application, the CDK inhibitor can be used for treating gastrointestinal side effects associated with the administration of the following chemotherapeutic agents: fluorouracil, oxaliplatin, irinotecan, docetaxel, gemcitabine, carboplatin, methotrexate, doxorubicin, cytarabine, vinorelbine and capecitabine, and any combination of the above.

In the present application, the chemotherapy may be administered continuously. For example, the chemotherapy may be administered continuously for 2 days, 3 days, 4 days, 5 days, 6 days, 7 days, 8 days, 9 days or more days. In some embodiments, the days for the continuous administration of the chemotherapy are no more than 7 days. For example, the chemotherapy may be administered continuously for 2 days, 3 days, 4 days, 5 days or 6 days. In the present application, the administration frequency of the chemotherapy may be once a day, twice a day, 3 times a day or others.

In the present application, the chemotherapy may be administered non-continuously. In the present application, the administration frequency of the chemotherapy may be once every two days, once every three days, twice every three days or others.

In the present application, the administration frequency of the chemotherapeutic agent may be once a day, and the administration may be continued for less than 7 days and/or no more than 7 days.

In the present application, the administration cycle of the chemotherapeutic agent may be 3 days, 5 days, 7 days, 2 weeks, 20 days, 1 month, 2 months or longer.

In the present application, one or more different chemotherapeutic agents may be used. In the present application, two or more different chemotherapeutic agents may be used in combination. In some embodiments, the CDK inhibitor may be used in combination with one or more other cancer therapies. The other cancer therapies may be common methods used for treating cancers in the art, e.g., cytotoxic anticancer agent, immunotherapeutic anticancer agent or hormone therapy anticancer agent. In accordance with the present application, the medicament used for treating a cancer may also be used in combination with radiotherapy or surgery. In some embodiments, in the case of the combination use of a CDK inhibitor and other anticancer agents, they can be administered to the subject at the same time, or separately administered at a certain interval.

### Gastrointestinal side effects

In the present application, the CDK inhibitor can prevent, relieve and/or treat gastrointestinal side effects associated with the administration of chemotherapy in a subject. In the present application, chemotherapy-associated gastrointestinal side effects may mean that the gastrointestinal side effects are caused by the administration of the chemotherapy and will occur or deteriorate after the administration of the chemotherapeutic agent. In the absence of prevention or treatment, the gastrointestinal side effects will occur or deteriorate 1 hour, 2 hours, 3 hours, 4 hours, 5 hours, 6 hours, 7 hours, 8 hours, 9 hours, 10 hours, 11 hours, 12 hours, 1 day, 2 days, 4 days, 7 days, 2 weeks, 3 weeks, 1 month, 2 months or longer after the administration of the chemotherapeutic agent.

In some embodiments, before administering the chemotherapeutic agent to the subject, the subject does not develop the gastrointestinal side effects; after administering the chemotherapeutic agent to the subject, the subject develops the gastrointestinal side effects.

In some embodiments, before administering the chemotherapeutic agent to the subject, the subject has developed the gastrointestinal side effects; after administering the chemotherapeutic agent to the subject, the severity of the gastrointestinal side effects in the subject increases.

In the present application, after the administration of the chemotherapeutic agent, the symptoms of gastrointestinal side effects in the subject may deteriorate by at least about 10%, e.g., by about 15%, about 20%, about 25%, about 30%, about 35%, about 40%, about 45%, about 50%, about 55%, about 60%, about 65%, about 70%, about 75%, about 80%, about 85%, about 90%, about 95%, about 99% or higher.

For example, in accordance with the criteria of NCI-CTCAE, after administering the chemotherapeutic agent to the subject, the severity of the gastrointestinal side effects (e.g., diarrhea or constipation) in the subject may increase from Grade 1 to Grade 2, from Grade 1 to Grade 3, from Grade 1 to Grade 4, from Grade 1 to Grade 5, from Grade 2 to Grade 3, from Grade 2 to Grade 4, from Grade 2 to Grade 5, from Grade 3 to Grade 4, from Grade 3 to Grade 5 or from Grade 4 to Grade 5. For example, in accordance with the method of Akinobu Kurita, after administering the chemotherapeutic agent to the subject, the scoring of constipation in the subject increases from Grade 0 to Grade 1, from Grade 0 to Grade 2, from Grade 0 to Grade 3, from Grade 1 to Grade 2, from Grade 1 to Grade 3 or from Grade 2 to Grade 3.

In some embodiments, the gastrointestinal side effects comprise gastric mucosal injury diseases and/or intestinal mucosal injury diseases.

In some embodiments, the gastrointestinal side effects comprise diarrhea, abdominal pain, nausea, vomiting, mucositis, loss of appetite, gastric ulcer, gastritis, constipation, enteritis, intestinal perforation, intestinal hemorrhage, ulcer and/or intestinal necrosis. In some embodiments, the gastrointestinal side effects comprise abnormal defecation. In some embodiments, the gastrointestinal side effects comprise diarrhea and/or constipation.

In some embodiments, the chemotherapy-associated gastrointestinal side effects comprise chemotherapy-associated gastric mucosal injury diseases and/or chemotherapy-associated intestinal mucosal injury diseases.

In some embodiments, the chemotherapy-associated gastrointestinal side effects comprise chemotherapy-associated diarrhea, chemotherapy-associated abdominal pain, chemotherapy-associated nausea, chemotherapy-associated vomiting, chemotherapy-associated mucositis, chemotherapy-associated loss of appetite, chemotherapy-associated gastric ulcer, chemotherapy-associated gastritis, chemotherapy-associated constipation, chemotherapy-associated enteritis, chemotherapy-associated intestinal perforation, chemotherapy-associated intestinal hemorrhage, chemotherapy-associated ulcer and/or chemotherapy-associated intestinal necrosis. In some embodiments, the chemotherapy-associated gastrointestinal side effects comprise chemotherapy-associated abnormal defecation. In some embodiments, the chemotherapy-associated gastrointestinal side effects comprise chemotherapy-associated diarrhea and/or chemotherapy-associated constipation.

In the present application, after administration of the CDK inhibitor of the present application, the severity of chemotherapy-associated gastrointestinal side effects in the subject is relieved. In the present application, the relief may generally mean that the occurrence or development of the gastrointestinal side effects in the subject is delayed. In the present application, after administration of the CDK inhibitor, the symptoms of gastrointestinal side effects in the subject may be ameliorated. In the present application, after administration of the CDK inhibitor, the symptoms of gastrointestinal side effects in the subject may be ameliorated by at least about 10%, e.g., by about 15%, about 20%, about 25%, about 30%, about 35%, about 40%, about 45%, about 50%, about 55%, about 60%, about 65%, about 70%, about 75%, about 80%, about 85%, about 90%, about 95%, about 99% or more.

For example, in accordance with the criteria of NCI-CTCAE, after administration of the CDK inhibitor, the symptoms of gastrointestinal side effects (e.g., diarrhea and/or constipation) in the subject may decrease from Grade 5 to Grade 4, from Grade 5 to Grade 3, from Grade 5 to Grade 2, from Grade 5 to Grade 1, from Grade 4 to Grade 3, from Grade 4 to Grade 2, from Grade 4 to Grade 1, from Grade 3 to Grade 2, from Grade 3 to Grade 1 or from Grade 2 to Grade 1.

Alternatively, in accordance with the method of Akinobu Kurita, after administration of the CDK inhibitor, the scoring of diarrhea in the subject may decrease from Grade 3 to Grade 2, from Grade 3 to Grade 1, from Grade 3 to Grade 0, from Grade 2 to Grade 1, from Grade 2 to Grade 0 or from Grade 1 to Grade 0.

In the present application, after administration of the CDK inhibitor, the symptoms of gastrointestinal side effects in the subject may be eliminated. However, the case that the gastrointestinal side effects recur or deteriorate again after stopping the administration of the CDK inhibitor may not be excluded.

### Preparation method, composition

In another aspect, the present application provides a method for preparing the above-mentioned compound of the present application, or a pharmaceutically acceptable salt, bioactive metabolite, solvate, hydrate, prodrug, racemate, enantiomer or stereoisomer thereof.

In another aspect, the present application provides a composition comprising the compound of the present application, or a pharmaceutically acceptable salt, bioactive metabolite, solvate, hydrate, prodrug, racemate, enantiomer or stereoisomer thereof.

In the present application, the composition may include a compound provided herein, such as a compound of formula (I), or an enantiomer, a mixture of enantiomers, a diastereomer, a mixture of two or more diastereomers, a tautomer, or a mixture of two or more tautomers thereof; or a pharmaceutically acceptable salt, solvate, hydrate or prodrug thereof.

In certain embodiments, the composition of the present application is a pharmaceutical composition. For example, it may contain one or more pharmaceutically acceptable carriers. For example, the pharmaceutically acceptable carrier may be selected from the group consisting of fillers, binders, disintegrants, buffers, preservatives, lubricants, flavoring agents, thickeners, colorants, and emulsifiers.

In the present application, many carriers and excipients may serve multiple functions, even within the same formulation.

The compositions provided herein can be formulated into a variety of dosage forms, including but not limited to dosage forms for oral administration. The compositions can also be formulated into modified release formulations, including delayed, extended, sustained, controlled, accelerated, rapid, targeted, programmed release and gastric retention dosage forms. These dosage forms can be prepared according to conventional methods and techniques known to those skilled in the art. See, for example, Remington: The Science and Practice of Pharmacy, supra; Modified-Release Drug Delivery Technology, 2nd ed.; Rathbone et al., Eds.; Drugs and the Pharmaceutical Sciences 184; CRC Press: Boca Raton, FL, 2008.

The composition provided herein can be provided in unit dosage form or multiple dosage form. As used herein, unit dosage form refers to a physical discrete unit suitable for supplying a patient, i.e., each unit contains a predetermined amount of calculated active agent, to produce the desired therapeutic effect alone or in combination with one or more additional units. For example, the unit dosage form can be a capsule, a tablet, a pill and the like, or a unit package suitable for parenteral administration. The unit dosage form can be administered in divided or multiple doses. Multiple dosage forms are multiple identical unit dosage forms packaged in a single container, administered in separate unit dosage forms. Examples of multiple dosage forms include, but are not limited to, sample bottles, tablets or capsule bottles.

The composition provided herein can be administered single or multiple doses at intervals. It should be understood that the exact dosage and duration of treatment can vary with the age, weight and condition of the subject being treated, and can be determined using known experimental protocols or by empirical speculation from in vivo or in vitro tests or diagnostic data. It should also be understood that for any particular individual, the specific dosage regimen should be adjusted at any time according to the needs of the subject and the professional judgment of the management/supervisor.

### Method, use, kit

In another aspect, the present application provides the use of the compound, or a pharmaceutically acceptable salt, bioactive metabolite, solvate, hydrate, prodrug, racemate, enantiomer or stereoisomer thereof, or the pharmaceutical composition described herein, in the preparation of a medicament for the prevention, alleviation, and/or treatment of chemotherapy-associated gastrointestinal side effects in a subject.

In another aspect, the present application provides a method for preventing, alleviating and/or treating chemotherapy-associated gastrointestinal side effects in a subject, comprising administering to the subject in need thereof the compound, or a pharmaceutically acceptable salt, bioactive metabolite, solvate, hydrate, prodrug, racemate, enantiomer or stereoisomer thereof, or the composition.

In another aspect, the present application provides the compound, or a pharmaceutically acceptable salt, bioactive metabolite, solvate, hydrate, prodrug, racemate, enantiomer or stereoisomer thereof, or the composition, for use in preventing, alleviating and/or treating chemotherapy-associated gastrointestinal side effects in a subject.

The term "prevention" as used herein generally refers to the prevention of occurrence, recurrence, or spread of diseases or one or more symptoms thereof. In the context of the present application, the "prevention" may be interchangeably used with the "preventive treatment". In some embodiments, the "prevention" generally refers to the treatment of providing a patient suffering from the diseases or disorders as described in the present application with the medicament in accordance with the present application with or without administration of other medicaments as described in the present application prior to the occurrence of any symptoms. In some embodiments, patients with a family history of a particular disease can be the candidates of a prevention program. In some embodiments, patients with a history of recurring symptoms are also potential objects of prevention.

The term "treatment" as used herein generally refers to eliminating or improving diseases or one or more symptoms associated with the diseases. In some embodiments, the treatment generally refers to eliminating or ameliorating a disease by administering one or more therapeutic agents to the patients suffering from the disease. In some embodiments, the "treatment" may be administering a medicament in the presence or absence of other therapeutic agent(s) after the occurrence of symptoms of a particular disease.

The term "subject" as used in this application generally refers to a human or non-human animal (including mammals) that needs to diagnose, prognose, improve, prevent, alleviate and/or treat a disease, particularly those subjects that need the compound or the composition to treat or prevent. In some embodiments, the subject may include a cancer patient. For example, the cancer patient may have been, is being and/or will be administered chemotherapy.

In some embodiments, the subject may be a human or a non-human mammal. The non-human mammals may include any mammalian species other than human, e.g., livestocks (e.g., cow, pig, sheep, chick, rabbit or horse), or rodents (e.g., rat and mouse), or primates (e.g., gorilla and monkey), or domestic animals (e.g., dog and cat). The "subject" may be male or female, and also may be at different ages.

The term "effective amount" as used herein generally refers to an amount of medicament capable of ameliorating or eliminating diseases or symptoms of the subject, or preventively inhibiting or prohibiting the occurrence of diseases or symptoms. The effective amount may be an amount of medicament capable of ameliorating one or more diseases or symptoms to some extent in the subject; an amount of medicament capable of partially or completely restoring one or more physiological or biochemical parameters associated with the causes of diseases or symptoms to normal; and/or an amount of medicament capable of reducing the possibility of the occurrence of diseases or symptoms.

In the present application, before the administration of the chemotherapy, for example, 0.5 hours, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours or longer before the administration of the chemotherapy, the compound or the composition can be administered to prevent, relieve and/or treat the occurrence or deterioration of the gastrointestinal side effects. For example, 0.5-12 hours before chemotherapy is administered, the compound or the composition is administered to prevent, alleviate and/or treat the generation or aggravation of gastrointestinal side effects.

In the present application, after the administration of the chemotherapy, for example, 0.5 hours, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours or longer after the administration of the chemotherapy, the compound or the composition can be administered to prevent, relieve and/or treat the occurrence or deterioration of the gastrointestinal side effects. For example, 0.5-12 hours after chemotherapy is administered, the compound or the composition is administered to prevent, alleviate and/or treat the generation or aggravation of gastrointestinal side effects.

In the present application, the administration site of the compound may be or not be the occurrence site of cancer or the potential metastatic site of cancer.

The compounds described herein may be administered in a manner known in the art, e.g., by injection (e.g., subcutaneous, intraperitoneal, intraarticular, intraarterial, intrathecal, intrasternal, intrathecal, intralesional, intracranial, intramuscular, intracutaneous and intravenous injection or infusion) or non-injection (e.g., oral, nasal, sublingual, vaginal, rectal, or topical administration). The compounds of the present application may be administered in a form of a pharmaceutical combination or a kit. In some embodiments, the compounds of the present application may be administered in the same administration route as that of the chemotherapy or in a different route.

In the present application, the drug and/or the compound may be formulated for oral administration. In the present application, the compound may preferentially act in the enteric cavity, or may preferentially get into the enteric cavity, rather than being exposed to the systemic circulation. The compound may be prepared as a dosage form suitable for being delivered into the enteric cavity or taking effects in the enteric cavity (e.g., the effects of preventing, relieving and/or treating gastrointestinal side effects). The compound can be administered in a route or manner that is suitable for delivering it into the enteric cavity or facilitating it to take effects in the enteric cavity, or auxiliary means such as devices can also be used to deliver the compound into the enteric cavity or make it to take effects in the enteric cavity. In the present application, the systemic medicament concentration of the compound is lower than the minimum inhibitory concentration (IC50).

In the present application, the drug and/or the compound may be prepared for gastrointestinal administration, e.g., powders, tablets, granules, capsules, solution, emulsion and/or suspension. In the present application, the medicament and/or the compound may be prepared as a dosage form suitable for translumenal administration, e.g., suppositories and/or drop pills. In the present application, the medicament and/or the compound may be prepared a dosage form suitable for being delivered to the gastrointestinal tract by an artificial auxiliary means, e.g., by intubation.

In the present application, the drug and/or the compound is prepared as a dosage form suitable for gastrointestinal exposure. The dosage form suitable for gastrointestinal exposure may be a dosage form suitable for being delivered to the gastrointestinal tract, a dosage form suitable for gastrointestinal administration, a dosage form suitable for translumenal administration, a dosage form suitable for oral administration and/or a dosage form suitable for being delivered to the gastrointestinal tract by an artificial auxiliary means.

In the present application, the compound may be administered at a dosage of about 0.01-1000 mg/kg, for example, about 10 - 1000 mg/kg, about 20 - 1000 mg /kg, about 30 - 1000 mg /kg or about 50 - 1000 mg/kg.

For example, the compound can be administered orally at a dosage of about 0.01-1000 mg/kg, for example, about 10 - 1000 mg/kg, about 20 - 1000 mg /kg, about 30 - 1000 mg/kg, or about 50 - 1000 mg/kg.

For example, the compound can be administered orally at a dosage of about 5 mg/kg, about 10 mg/kg, about 15 mg/kg, about 20 mg/kg, about 25 mg /kg, about 30 mg/kg, about 50 mg/kg, about 75 mg /kg, about 100 mg/kg, about 200 mg/kg.

A certain dosage may be administered intervals in many times, e.g. once a day, twice a day or more times a day, once a week, once every two weeks, once every three weeks, once a month or once every two or more months. In some embodiments, the administration dosage may vary with the course of treatment. For example, in some embodiments, the initial administration dosage may be higher than the subsequent administration dosage. In some embodiments, the administration dosage is adjusted in the course of treatment in accordance with the response of the administration object. When being used to improve the conditions of the subject, the CDK inhibitor of the present application may be administered at a maintenance dose as needed. Then, the dosage or frequency of administration or both can be reduced to a level for maintaining the improved conditions when the symptoms are ameliorated to the desired level. In some embodiments, the medicament may be administered at intervals depending on the conditions of disease in the subject.

The compounds provided in this application may also be used in combination with other therapeutic agent/therapeutic agent combinations that may be used to treat and/or prevent the diseases or conditions described in the present invention.

In the present application, the term "combination" includes the use of more than one therapy (for example, one or more preventive agents and/or therapeutic agents). However, the use of the term " combination " does not limit the order in which therapy (for example, preventive agent and/or therapeutic agent) is applied to the subject suffering from a disease or condition. The first therapy (for example, preventive agent or therapeutic agent, such as compound provided by the invention) may be administered to the subject before, concurrently with, or after the second therapy ( for example, preventive agent or therapeutic agent), for example, (5 minutes, 15 minutes, 30 minutes, 1 hour, 2 hours, 6 hours, 12 hours, 24 hours, 48 hours, 72 hours, 96 hours, 1 week, 2 weeks, 3 weeks, 6 weeks, 8 weeks or 12 weeks). The present application also encompasses triple combination therapies.

The present application is independent of the administration route of the second therapy. In certain embodiments, the compound provided by the present application is orally administered. Therefore, according to the embodiment, the compound provided by the present invention is orally administered, and the second therapy can be administered orally, parenterally, intraperitoneally, intravenously, intraarterially, transdermally, subcutaneously, sublingually, intramuscularly, rectally, buccally, intranasally, liposomes, inhaled, vaginally, intraocularly, locally delivered by a catheter or stent, intrafatally, intraarticularly, intrathecally. In some embodiments, the compound provided by the present invention and the second therapy are orally administered by the same mode of administration. In certain embodiments, the compound provided by the present application is administered orally by a mode of administration such as oral administration, and the second agent (anticancer agent) is administered by another mode of administration such as parenteral administration.

On the other hand, the present application provides a method for inhibiting the activity of cyclin-dependent kinase (CDK), comprising reacting CDK with an effective amount of a compound of formula (I), or a pharmaceutically acceptable salt, bioactive metabolite, solvate, hydrate, prodrug, racemate, enantiomer or stereoisomer thereof.

On the other hand, the present application provides a kit that, when used by a medical practitioner, can simplify administering an appropriate amount of the compound provided by the present invention as an active ingredient to a subject. In certain embodiments, the kit provided by the present application includes a container and a dosage form of the compound provided by the present invention. The kit of the present application may also include instructions for use.

The present application provided herein may also include a pharmaceutically acceptable carrier that can be used to administer one or more active ingredients. For example, if the active ingredient is provided in solid form and must be reconstituted for parenteral administration, the kit may include a sealed container of a suitable carrier in which the active ingredient can be dissolved to form a sterile solution free of particles that is suitable for parenteral administration.

Without intending to be bound by any theory, the following examples are merely intended to illustrate the compounds, preparation methods and uses of the present application, and are not intended to limit the scope of the present invention.

### EXAMPLES

### Example 1 Synthesis and structural characterization of compound 1 of the present application

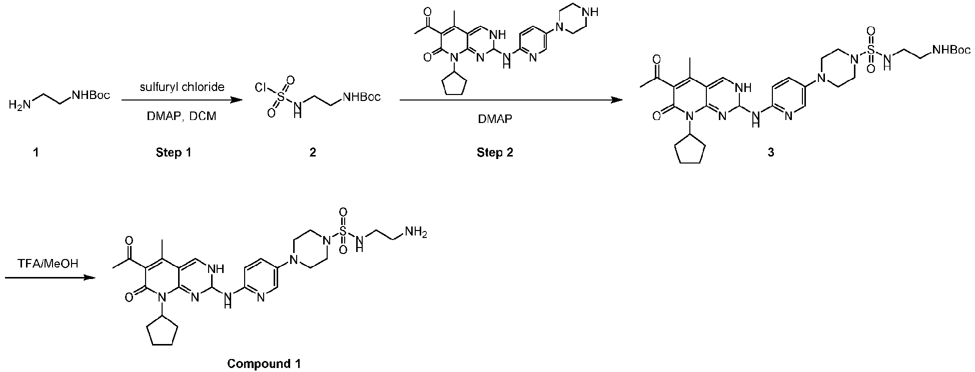

### Step 1: Synthesis of (tert-butyl (2)-(chlorosulfonyl) aminoethyl) carbamate

At -70°C, a solution of DMAP (63.24 g) and N-Boc-1,2-ethylenediamine (91.22 g) in dichloromethane (200 mL) was added to a solution of sulfonyl chloride (69.86 g) in dichloromethane (800 mL). The reaction mixture was warmed to 15 °C and stirred for 2 hours to afford a dichloromethane solution of tert-butyl (2-(chlorosulfonyl)aminoethyl)carbamate, which was used directly in the next step.

### Step 2: Synthesis of Intermediate 3

To a dichloromethane (400 mL) solution of palbociclib (50 g) and DMAP (75.89 g), a dichloromethane solution of tert-butyl (2-(chlorosulfonyl)aminoethyl)carbamate (obtained from Step 1) was added dropwise. The reaction mixture was stirred at 40 °C for 16 hours. The mixture was diluted with dichloromethane (500 mL), and washed twice with water (200 mL). The organic layer was separated, dried, and the solvent was removed under reduced pressure to yield a yellow solid. The crude product was slurried in a dichloromethane/ethyl acetate mixture (10/1, 1 L), filtered, and dried under reduced pressure to afford **Intermediate 3** (61 g, 87.97% yield) as a yellow solid. ¹H NMR (400 MHz, DMSO-d6) δ: 10.21 (s, 1H), 8.97 (s, 1H), 8.09 (d, *J* = 2.9 Hz, 1H), 7.89 (d, *J* = 9.0 Hz, 1H), 7.53 (dd, *J* = 2.9, 9.1 Hz, 1H), 7.40 (t, *J =* 5.7 Hz, 1H), 6.85 (br t, *J =* 5.4 Hz, 1H), 5.91 - 5.76 (m, 1H), 3.22 (br dd, *J =* 5.4, 15.6 Hz, 8H), 3.08 - 2.99 (m, 2H), 2.99 - 2.91 (m, 2H), 2.43 (s, 3H), 2.31 (s, 3H), 2.28 - 2.19 (m, 2H), 1.96 - 1.84 (m, 2H), 1.83 - 1.70 (m, 2H), 1.65 - 1.52 (m, 2H), 1.37 (s, 9H). MS = 670.4 (M+1).

### Step 3: Synthesis of Compound 1

Intermediate 3 (61 g) was dissolved in dichloromethane (1 L), and a trifluoroacetic acid/methanol solution (v/v = 1:1, 150 g, 4.1 mL) was added. The mixture was stirred at 40 °C for 28 hours. The solvent was removed under reduced pressure to afford **Compound 1** (43 g). ¹H NMR (400 MHz, DMSO-d6) δ: 11.61 (br s, 1H), 9.05 (s, 1H), 8.30 (br, 2H), 8.16 (dd, *J* = 9.60, 2.32 Hz, 1H), 8.06 (d, *J* = 2.72 Hz, 1H), 7.88 (t, *J =* 5.76 Hz, 1H), 7.82 (d, *J =* 9.28 Hz, 2H), 5.84 (quin, *J* = 8.80 Hz, 1H), 3.34 (m, 4H), 3.27 (m, 4H), 3.22 (m, 2H), 2.93 (m, 2H), 2.44 (s, 3H), 2.35 (s, 3H), 2.22 (m, 2H), 1.97 (m, 2H), 1.81 (m, 2H), 1.60 (m, 2H).

MS analysis showed a molecular ion peak at m/z 572.27 [M+H⁺].

### Example 2 Synthesis of Compounds 2-6 of the Present Application

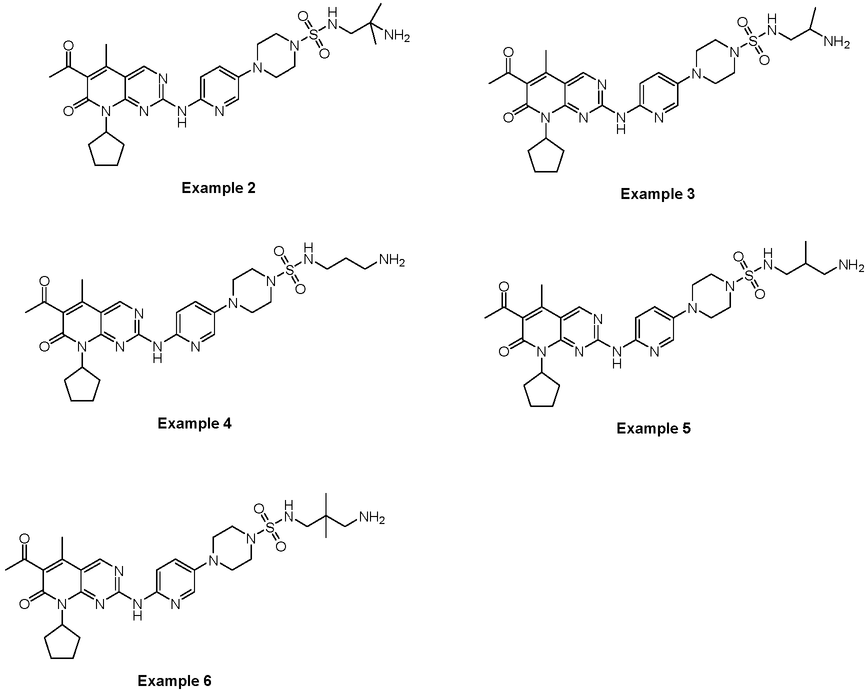

The synthetic routes for Compounds 2-6 are the same as that of Compound 1. The mass spectrometry results are shown in Table 1.

**Table 1. Mass spectrometry results of compounds 2-6**

| Compound No. | [M+H⁺] mass spectrometry results |
|---|---|
| Compound 2 | 598.75 |
| Compound 3 | 584.72 |
| Compound 4 | 584.72 |
| Compound 5 | 598.75 |
| Compound 6 | 612.78 |

Example 3 In vitro enzyme inhibitory activity of the compounds described of the present application

The KinaseProfile radiometric protein kinase assay was used to evaluate the inhibitory activity of the compounds against CDK2/cyclin E, CDK4/cyclin D, CDK6/cyclin D, CDK7/cyclin H and CDK9/cyclin T.

The compounds were prepared in 100% DMSO at a final 50× stock concentration. The working stock solution was added as the first component to the assay wells. CDK2/cyclin E, CDK4/cyclin D, CDK6/cyclin D, CDK7/cyclin H, and CDK9/cyclin T were added in their respective kinase buffers: (CDK4: 8 mM MOPS pH 7.0, 0.2 mM EDTA, 0.03% BSA, 0.03% Tween 20, 20 mM DTT, and 0.2 mg/ml Rb fragment, 10 mM Magnesium acetate; CDK2, CDK6: 8 mM MOPS pH 7.0, 0.2 mM EDTA, 0.1 mg/mL histone H1, 10 mM Magnesium acetate; CDK7: 8 mM MOPS pH 7.0, 0.2 mM EDTA, 500 µM peptide, 10 mM Magnesium acetate; CDK9: 8 mM MOPS pH 7.0, 0.2 mM EDTA, 100 µM KTFCGTPEYLAPEVRREPRILSEEEQEMFRDFDYIADWC, 10 mM Magnesium acetate) and [γ- ³³ P-ATP] was included in the reaction mixture. The reaction was initiated by the addition of the Mg/ATP mixture and incubated at room temperature for 40 minutes. The reaction was terminated by the addition of 0.5% phosphoric acid. Then, 10 µL of the reaction mixture was spotted onto P30 filter pads, washed four times (1 minute each) with 0.425% phosphoric acid, followed by a single wash with methanol. The pads were then dried and counted. The data were recorded, inhibition rates were calculated, and IC₅₀ values were determined accordingly. Table 2 summarizes the in vitro enzymatic inhibitory activity of the compounds of the present application.

**Table 2 In vitro enzymatic inhibitory activity of the compounds of the present invention**

| Example | Protein kinase activity IC50 (nM) | | | | |
|---|---|---|---|---|---|
| | CDK2/ cyclin E | CDK4/ cyclin D | CDK6/ cyclin D | CDK7/ cyclin H | CDK9/ cyclin T |
| Compound 1 | >10000 | <50 | <50 | > 10000 | >1000 |
| Compound 2 | >10000 | <50 | <50 | >3000 | >1000 |
| Compound 3 | >10000 | <50 | <100 | > 10000 | >3000 |
| Compound 4 | >3000 | <50 | <50 | >3000 | >3000 |
| Compound 5 | >3000 | <100 | <50 | > 10000 | >3000 |
| Compound 6 | >10000 | <50 | <100 | >3000 | >1000 |

### Example 4 Cellular activity assay of the compounds of the present application

### 4.1 Cell viability assay

The cell viability of MCF-7 human breast cancer cells under standard culture conditions was assessed using theCELLTITER-GLO assay. MCF-7 cells were seeded at 3,000 cells per well in 96-well plates and incubated overnight in a CO₂ incubator. The next day, serial dilutions of Compound 1 at various concentrations were added to the wells. The plates were returned to the incubator and cultured for 7 days. After 7 days, the plates were equilibrated at room temperature for 30 minutes. Subsequently, 100 µL of CELLTITER-GLO detection solution was added. The cells were mixed on an orbital shaker for 5 min and then incubated for additional 10 min. The plate was read with an ENVISION reader. Data were processed and analyzed to determine the IC₅₀ values. The experimental results are summarized in **Table 3.**

**Table 3. Cell Viability Assessment of the Compounds of the Present Invention**

| Example | Relative IC 50 (µM) | Absolute IC 50 (µM) |
|---|---|---|
| Compound 1 | <1 | <1 |
| Compound 2 | <1 | <0.5 |
| Compound 3 | <5 | <10 |
| Compound 4 | <1 | <1 |
| Compound 5 | <10 | <10 |
| Compound 6 | <1 | <1 |

The results demonstrated that the compounds of the present application exhibit significant inhibitory activity against MCF-7 cell viability.

### 4.2 Cell apoptosis assay

The Caspase-Glo^{®} 3/7 assay was employed to evaluate the protective effect of the compounds of the present invention against 5-fluorouracil (5-FU)-induced apoptosis. Human colorectal cancer COLO205 cells were cultured in RPMI 1640 medium (Invitrogen) in an incubator at 37°C with 5% CO₂.

COLO205 cells were seeded in 96-well plates at a density of 4,000 cells per well and incubated overnight. On the following day, serially diluted concentrations of Compound 1 were added to the wells and incubated for 16 hours. Afterward, 5-FU was added and the cells were incubated for an additional 48 hours. Subsequently, Caspase-Glo^{®} 3/7 reagent (100 µL) was added to each well. The cells were mixed on an orbital shaker for 10 min and incubated at room temperature for 2 h. After an additional 10-minute incubation, the plate was measured using the Envision instrument and the data was processed. Relief rate (%) = (5-FU group - CDK group) / 5-FU group × 100%. The experimental results are shown in **Table 4.**

**Table 4 Protective effect of the compounds of the present invention against 5-FU-induced cell apoptosis**

| Example | Concentration (µM) | Relief rate of 5-FU -induced cell apoptosis (%) |
|---|---|---|
| Compound 1 | 0.1 | 30 |
| | 1 | 60 |
| | 5 | 80 |
| | 25 | 40 |
| Compound 2 | 1 | 40 |
| | 5 | 50 |
| | 10 | 70 |
| | 50 | 40 |
| Compound 3 | 0.1 | 20 |
| | 1 | 40 |
| | 5 | 40 |
| | 25 | 30 |
| Compound 4 | 10 | 40 |
| | 25 | 50 |
| | 50 | 50 |
| | 100 | 30 |
| Compound 5 | 0.5 | 10 |
| | 5 | 50 |
| | 50 | 40 |
| | 100 | 30 |
| Compound 6 | 0.1 | 20 |
| | 1 | 40 |
| | 5 | 60 |
| | 25 | 70 |

As shown in Table 4, the compounds of the present application significantly protect COLO205 cells from 5-FU-induced damage.

### Example 5 In vivo pharmacodynamic activity of the compounds of the present application

### 5.1. 5-FU-induced diarrhea model

The 5-FU-induced diarrhea model was used as an experimental model of the present invention to evaluate the in vivo activity of the compounds.

### 5-FU 1-day model

Balb/c mice (20-25 g, 7-8 weeks old, Jihui) were acclimated for one week and then randomly assigned to 3 groups (n = 10 per group): control group, 5-FU group, and CDK inhibitor group. The dosing schedule and dosages are shown in Table 5. The control group received the same vehicle as the CDK inhibitor group by oral gavage, followed by injection of the same vehicle as the 5-FU group. The 5-FU group received the same vehicle by oral gavage as the CDK inhibitor group, followed by 5-FU injection. The CDK inhibitor group received oral administration of the CDK inhibitor, followed by 5-FU injection. From day 1 to day 7, the severity of diarrhea was assessed for each mouse according to the diarrhea scoring system.

### 5-FU 3-day model

Balb/c mice (20-25 g, 7-8 weeks old, Jihui) were acclimated for one week and then randomly assigned to 3 groups (n = 10 per group): control group, 5-FU group, and CDK inhibitor group. As shown in Table 6, the control group received the same vehicle as the CDK inhibitor group by oral gavage, followed by injection of the same vehicle as the 5-FU group, for 3 consecutive days. The 5-FU group received the same vehicle by oral gavage as the CDK inhibitor group, followed by 5-FU injection, for 3 consecutive days. The CDK inhibitor group received oral administration of the CDK inhibitor, followed by 5-FU injection, for 3 consecutive days. The dosing schedule and frequency are shown in Table 6. From Day 1 to Day 7, diarrhea severity was assessed daily for each mouse according to the diarrhea scoring system.

The diarrhea scoring system was based on the method by Akinobu Kurita (Cancer Chemother Pharmacol 2000; 46: 211-20.), Grade 0: normal stool; Grade 1: mild diarrhea, slightly soft stool and wet stool; Grade 2: moderate diarrhea, loose stool with slight perianal staining; Grade 3: severe diarrhea, loose watery stool with severe perianal staining.

The diarrhea relief rate (%) was calculated as follows: Diarrhea relief rate (%) = (Diarrhea score of 5-FU group - Diarrhea score of CDK inhibitor group) / Diarrhea score of 5-FU group × 100%.

**Table 5. Pharmacodynamic activity of the compounds in the 5-FU 1-day model**

| Example | CDK inhibitor dose (mg/kg) | CDK inhibitor administration time | 5- FU dose (mg/kg) | 5- FU dosing frequency and duration | Relief rate (%) |
|---|---|---|---|---|---|
| Compound 1 | 100 | Administer 8 hours before 5-FU and 4 hours after 5-FU | 175 | Once a day, 1 day | 75 |
| | 50 | Administer 8 hours before 5-FU and 4 hours after 5-FU | 175 | Once a day, 1 day | 60 |
| | 5 | Administer 8 hours before 5-FU and 4 hours after 5-FU | 175 | Once a day, 1 day | 50 |
| | 25 | Administer 12 hours before 5-FU | 175 | Once a day, 1 day | 85 |
| | 10 | Administer 6 hours before and 6 hours after 5-FU | 175 | Once a day, 1 day | 40 |
| | 75 | Administer 6 hours before and 6 hours after 5-FU | 175 | Once a day, 1 day | 50 |
| Compound 2 | 25 | Administer 12 hours before and 12 hours after 5-FU | 175 | Once a day, 1 day | 40 |
| | 100 | Administered 24 hours before 5-FU and 0 hours after 5-FU | 175 | Once a day, 1 day | 40 |
| | 25 | Administer 8 hours before 5-FU and 4 hours after 5-FU | 175 | Once a day, 1 day | 60 |
| | 50 | Administer 6 hours before and 6 hours after 5-FU | 175 | Once a day, 1 day | 60 |
| Compound 3 | 10 | Administer 10 hours before 5-FU and 2 hours after 5-FU | 175 | Once a day, 1 day | 50 |
| | 25 | Administer 2 hours before 5-FU and 10 hours after 5-FU | 175 | Once a day, 1 day | 40 |
| | 150 | Administer 12 hours before 5-FU and 0 hours after 5-FU | 175 | Once a day, 1 day | 50 |
| | 25 | Administer 8 hours before 5-FU and 4 hours after 5-FU | 175 | Once a day, 1 day | 60 |
| Compound 4 | 10 | Administer 8 hours before 5-FU and 4 hours after 5-FU | 175 | Once a day, 1 day | 25 |
| | 100 | Administer 8 hours before 5-FU and 4 hours after 5-FU | 175 | Once a day, 1 day | 25 |
| | 25 | Administer 6 hours before and 6 hours after 5-FU | 175 | Once a day, 1 day | 80 |
| | 50 | Administer 12 hours before 5-FU | 175 | Once a day, 1 day | 70 |
| Compound 5 | 10 | Administer 12 hours before 5-FU and 0 hours after 5-FU | 175 | Once a day, 1 day | 25 |
| | 25 | Administer 6 hours before and 6 hours after 5-FU | 175 | Once a day, 1 day | 40 |
| | 50 | Administer 8 hours before 5-FU and 4 hours after 5-FU | 175 | Once a day, 1 day | 30 |
| | 100 | Administer 4 hours before 5-FU and 8 hours after 5-FU | 175 | Once a day, 1 day | 40 |
| Compound 6 | 25 | Administer 8 hours before 5-FU and 4 hours after 5-FU | 175 | Once a day, 1 day | 75 |
| | 50 | Administer 6 hours before and 6 hours after 5-FU | 175 | Once a day, 1 day | 80 |
| | 10 | Administer 10 hours before 5-FU and 2 hours after 5-FU | 175 | Once a day, 1 day | 60 |
| | 100 | Administer 12 hours before 5-FU | 175 | Once a day, 1 day | 50 |

**Table 6 Pharmacodynamic activity of the compounds in the 5-FU 3-day model**

| Example | CDK inhibitor dose (mg/kg) | CDK inhibitor administration time | 5-FU Dose (mg/kg) | 5-FU dosing frequency and duration | Relief rate (%) |
|---|---|---|---|---|---|
| Compound 1 | 100 | Twice a day for 3 days | 60 | Once a day for 3 days | 50 |
| | 50 | Twice a day for 3 days | 60 | Once a day for 3 days | 40 |
| | 25 | Twice a day for 3 days | 60 | Once a day for 3 days | 60 |
| | 100 | Twice a day for 3.5 days | 60 | Once a day for 3 days | 40 |
| | 10 | Twice a day for 3.5 days | 60 | Once a day for 3 days | 40 |
| | 75 | Twice a day for 4 days | 60 | Once a day for 3 days | 55 |
| Compound 2 | 10 | Twice a day for 3 days | 60 | Once a day for 3 days | 25 |
| | 25 | Twice a day for 3 days | 60 | Once a day for 3 days | 40 |
| | 50 | Twice a day for 3 days | 60 | Once a day for 3 days | 60 |
| | 100 | Twice a day for 3.5 days | 60 | Once a day for 3 days | 50 |
| | 25 | Twice a day for 3.5 days | 60 | Once a day for 3 days | 50 |
| | 50 | Twice a day for 4 days | 60 | Once a day for 3 days | 40 |
| Compound 3 | 10 | Twice a day for 3 days | 60 | Once a day for 3 days | 30 |
| | 50 | Twice a day for 3 days | 60 | Once a day for 3 days | 40 |
| | 100 | Twice a day for 3 days | 60 | Once a day for 3 days | 70 |
| | 10 | Twice a day for 3.5 days | 60 | Once a day for 3 days | 40 |
| | 50 | Twice a day for 3.5 days | 60 | Once a day for 3 days | 40 |
| | 5 | Twice a day for 4 days | 60 | Once a day for 3 days | 25 |
| Compound 4 | 5 | Twice a day for 3 days | 60 | Once a day for 3 days | 30 |
| | 50 | Twice a day for 3 days | 60 | Once a day for 3 days | 60 |
| | 100 | Twice a day for 3 days | 60 | Once a day for 3 days | 40 |
| | 10 | Twice a day for 3.5 days | 60 | Once a day for 3 days | 50 |
| | 25 | Twice a day for 3.5 days | 60 | Once a day for 3 days | 55 |
| | 10 | Twice a day for 4 days | 60 | Once a day for 3 days | 40 |
| Compound 5 | 10 | Twice a day for 3 days | 60 | Once a day for 3 days | 40 |
| | 50 | Twice a day for 3 days | 60 | Once a day for 3 days | 40 |
| | 100 | Twice a day for 3 days | 60 | Once a day for 3 days | 50 |
| | 10 | Twice a day for 3.5 days | 60 | Once a day for 3 days | 75 |
| | 60 | Twice a day for 3.5 days | 60 | Once a day for 3 days | 50 |
| | 30 | Twice a day for 4 days | 60 | Once a day for 3 days | 30 |
| Compound 6 | 10 | Twice a day for 3 days | 60 | Once a day for 3 days | 30 |
| | 30 | Twice a day for 3 days | 60 | Once a day for 3 days | 50 |
| | 100 | Twice a day for 3 days | 60 | Once a day for 3 days | 60 |
| | 20 | Twice a day for 3.5 days | 60 | Once a day for 3 days | 50 |
| | 80 | Twice a day for 3.5 days | 60 | Once a day for 3 days | 60 |
| | 50 | Twice a day for 4 days | 60 | Once a day for 3 days | 75 |

The results shown in Tables 5 and 6 demonstrate that, compared with the 5-FU group, the diarrhea scores of animals treated with the compounds of the present application were significantly reduced, indicating that the compounds of the present application effectively alleviate 5-FU-induced diarrhea.

### 5.2. Irinotecan-induced diarrhea and/or constipation model

Irinotecan-induced diarrhea and/or constipation models were employed as additional in vivo experimental models to evaluate the activity of the compounds of the present application.

### Irinotecan -induced diarrhea model

Balb/c mice (20-25 g, 7-8 weeks old, Jihui) were acclimated for one week and then randomly assigned to 3 groups (n = 10 per group): control group, irinotecan group, and CDK inhibitor group. The dosing schedule and dosages are shown in Table 7. Control group: received the same vehicle as the CDK inhibitor group by oral gavage, followed by injection of the same vehicle as the irinotecan group; irinotecan group: received the same vehicle by oral gavage as the CDK inhibitor group, followed by injection of irinotecan; CDK inhibitor group: received oral administration of the CDK inhibitor, followed by injection of irinotecan.

In the irinotecan group, approximately 6 to 9 out of 10 mice developed diarrhea. From Day 1 to Day 7, the severity of diarrhea in each mouse was evaluated using the following scoring criteria:
The diarrhea scoring system was based on the method by Akinobu Kurita (Cancer Chemother Pharmacol 2000; 46: 211-20.), Grade 0: normal stool; Grade 1: mild diarrhea, slightly soft stool and wet stool; Grade 2: moderate diarrhea, loose stool with slight perianal staining; Grade 3: severe diarrhea, loose watery stool with severe perianal staining. Calculate the diarrhea Relief rate of compound treatment. Relief rate (%) = (diarrhea score of irinotecan group -diarrhea score of CDK inhibitor group)/ diarrhea score of irinotecan group × 100%.

As shown in Table 7, the results demonstrate that the diarrhea scores in the compound-treated group were significantly reduced compared to the irinotecan group, indicating that the compounds of the present invention exert a protective effect against irinotecan-induced diarrhea.

**Table 7 Pharmacodynamic activity of the compounds in the irinotecan-induced diarrhea model**

| Example | CDK inhibitor dose (mg/ kg) | CDK inhibitor administration time | Irinotecan dose (mg/kg) | Irinotecan dosing frequency and duration | Relief rate (%) |
|---|---|---|---|---|---|
| Compound 1 | 5 | Administer 4 hours before and 8 hours after irinotecan | 230 | Once a day, 1 day | 30 |
| | 50 | Administer 4 hours before and 8 hours after irinotecan | 230 | Once a day, 1 day | 37.5 |
| | 100 | Administer 4 hours before and 8 hours after irinotecan | 230 | Once a day, 1 day | 30 |
| | 10 | Administer 6 hours before and 6 hours after irinotecan | 230 | Once a day, 1 day | 33.3 |
| | 25 | Administer 6 hours before and 6 hours after irinotecan | 230 | Once a day, 1 day | 50 |
| | 100 | Administer 6 hours before and 6 hours after irinotecan | 230 | Once a day, 1 day | 20 |
| Compound 2 | 25 | Administer 4 hours before and 8 hours after irinotecan | 230 | Once a day, 1 day | 70 |
| | 50 | Administer 8 hours before irinotecan and 4 hours after irinotecan | 230 | Once a day, 1 day | 75 |
| | 25 | Administer 6 hours before and 6 hours after irinotecan | 230 | Once a day, 1 day | 60 |
| | 100 | Administer 12 hours before irinotecan | 230 | Once a day, 1 day | 50 |
| Compound 3 | 5 | Administer 4 hours before and 8 hours after irinotecan | 230 | Once a day, 1 day | 25 |
| | 50 | Administer 4 hours before and 8 hours after irinotecan | 230 | Once a day, 1 day | 70 |
| | 100 | Administer 6 hours before and 6 hours after irinotecan | 230 | Once a day, 1 day | 50 |
| | 25 | Administer 10 hours before and 2 hours after irinotecan | 230 | Once a day, 1 day | 60 |
| Compound 4 | 25 | Administer 2 hours before and 10 hours after irinotecan | 230 | Once a day, 1 day | 30 |
| | 50 | Administer 4 hours before irinotecan and 8 hours after irinotecan | 230 | Once a day, 1 day | 60 |
| | 50 | Administer 6 hours before irinotecan and 6 hours after irinotecan | 230 | Once a day, 1 day | 60 |
| | 150 | Administer 10 hours before irinotecan | 230 | Once a day, 1 day | 45 |
| Compound 5 | 5 | Administer 4 hours before irinotecan and 8 hours after irinotecan | 230 | Once a day, 1 day | 25 |
| | 25 | Administer 6 hours before irinotecan and 6 hours after irinotecan | 230 | Once a day, 1 day | 50 |
| | 50 | Administer 8 hours before irinotecan and 4 hours after irinotecan | 230 | Once a day, 1 day | 50 |
| | 100 | Administer 10 hours before irinotecan and 2 hours after irinotecan | 230 | Once a day, 1 day | 35 |
| | 25 | Administer 6 hours before irinotecan and 6 hours after irinotecan | 230 | Once a day, 1 day | 60 |
| | 10 | Administer 4 hours before irinotecan and 8 hours after irinotecan | 230 | Once a day, 1 day | 50 |
| | 100 | Administer 12 hours before irinotecan | 230 | Once a day, 1 day | 40 |

### Irinotecan-induced constipation model

Sprague-Dawley rats (180-200 g, 7-8 weeks old, Jihui) were acclimated for 7 days and randomly divided into three groups: control group, irinotecan group, and CDK inhibitor group, with 10 rats per group. The dosing schedule and administration details are provided in Table 8. The control group received the same vehicle orally as the CDK inhibitor group, followed by an injection of the same vehicle as the irinotecan group; the irinotecan group received the same oral vehicle as the CDK inhibitor group, followed by an injection of irinotecan; the CDK inhibitor group received the CDK inhibitor orally, followed by an injection of irinotecan.

In the irinotecan group, approximately 5 to 7 out of 10 rats began to exhibit constipation symptoms starting from Day 2. From Day 2 to Day 7, the degree of constipation was assessed for each rat based on the following score:
**Constipation assessment:** Feces excreted within a 3-hour period were collected and evaluated for morphology and weight using an electronic balance. Stool reduction rate was calculated according to the method of Ji Eun Kim (Lab Anim Res. 2016 Dec; 32(4): 231-240). Stool reduction rate (%) = (Control group - Irinotecan or CDK inhibitor group) / Control group × 100%. The constipation relief rate of the CDK inhibitor was calculated as follows: Relief rate (%) = (CDK inhibitor group stool reduction rate - Irinotecan group stool reduction rate) / Irinotecan group stool reduction rate × 100%. The results are shown in Table 8.

**Table 8 Pharmacodynamic Effects of the Compounds of the Present Invention in an Irinotecan-Induced Constipation Model**

| Example | CDK inhibitor dose (mg/ kg) | CDK inhibitor administration time | Irinotecan dose (mg/kg) | Irinotecan dosing frequency and duration | Relief rate (%) |
|---|---|---|---|---|---|
| Compound 1 | 2.5 | Administer 4 hours before irinotecan and 8 hours after irinotecan | 260 | Once a day, 1 day | 20 |
| | 25 | Administer 4 hours before irinotecan and 8 hours after irinotecan | 260 | Once a day, 1 day | 75 |
| | 100 | Administer 4 hours before irinotecan and 8 hours after irinotecan | 260 | Once a day, 1 day | 40 |
| | 5 | Administer 6 hours before irinotecan and 6 hours after irinotecan | 260 | Once a day, 1 day | 30 |
| | 50 | Administer 6 hours before irinotecan and 6 hours after irinotecan | 260 | Once a day, 1 day | 50 |
| | 100 | Administer 6 hours before irinotecan and 6 hours after irinotecan | 260 | Once a day, 1 day | 40 |
| Compound 2 | 5 | Administer 4 hours before irinotecan and 8 hours after irinotecan | 260 | Once a day, 1 day | 25 |
| | 50 | Administer 4 hours before irinotecan and 8 hours after irinotecan | 260 | Once a day, 1 day | 80 |
| | 100 | Administer 6 hours before irinotecan and 6 hours after irinotecan | 260 | Once a day, 1 day | 80 |
| | 150 | Administer 6 hours before irinotecan and 6 hours after irinotecan | 260 | Once a day, 1 day | 60 |
| Compound 3 | 50 | Administer 4 hours before irinotecan and 8 hours after irinotecan | 260 | Once a day, 1 day | 60 |
| | 50 | Administer 6 hours before irinotecan and 6 hours after irinotecan | 260 | Once a day, 1 day | 70 |
| | 25 | Administer 8 hours before irinotecan and 4 hours after irinotecan | 260 | Once a day, 1 day | 50 |
| | 25 | Administer 12 hours before irinotecan and 0 hours after irinotecan | 260 | Once a day, 1 day | 30 |
| Compound 4 | 10 | Administer 4 hours before irinotecan and 8 hours after irinotecan | 260 | Once a day, 1 day | 40 |
| | 25 | Administer 6 hours before irinotecan and 6 hours after irinotecan | 260 | Once a day, 1 day | 50 |
| | 50 | Administer 6 hours before irinotecan and 6 hours after irinotecan | 260 | Once a day, 1 day | 70 |
| | 100 | Administer 8 hours before irinotecan and 4 hours after irinotecan | 260 | Once a day, 1 day | 40 |
| | 100 | Administer 12 hours before irinotecan | 260 | Once a day, 1 day | 30 |
| Compound 5 | 5 | Administer 4 hours before irinotecan and 8 hours after irinotecan | 260 | Once a day, 1 day | 25 |
| | 50 | Administer 4 hours before irinotecan and 8 hours after irinotecan | 260 | Once a day, 1 day | 80 |
| | 100 | Administer 4 hours before irinotecan and 8 hours after irinotecan | 260 | Once a day, 1 day | 50 |
| | 25 | Administer 6 hours before irinotecan and 6 hours after irinotecan | 260 | Once a day, 1 day | 40 |
| | 50 | Administer 8 hours before irinotecan and 4 hours after irinotecan | 260 | Once a day, 1 day | 60 |
| | 100 | Administer 10 hours before irinotecan and 2 hours after irinotecan | 260 | Once a day, 1 day | 40 |
| Compound 6 | 10 | Administer 4 hours before irinotecan and 8 hours after irinotecan | 260 | Once a day, 1 day | 40 |
| | 25 | Administer 4 hours before irinotecan and 8 hours after irinotecan | 260 | Once a day, 1 day | 75 |
| | 25 | Administer 6 hours before irinotecan and 6 hours after irinotecan | 260 | Once a day, 1 day | 60 |
| | 50 | Administer 6 hours before irinotecan and 6 hours after irinotecan | 260 | Once a day, 1 day | 75 |
| | 150 | Administer 12 hours before irinotecan | 260 | Once a day, 1 day | 25 |

The results shown in Table 8 demonstrate that the compounds of the present invention exhibit a significant alleviating effect on irinotecan-induced constipation.

### Example 6 Single-Dose Intravenous and Oral Pharmacokinetic Study in Mice

Twenty-seven male Balb /c mice were randomly divided into 3 groups, 9 mice in each group. Group 1: mice received an intravenous (IV) dose of Compound 1 at 5 mg/kg with a dosing volume of 5 mL/kg. Blood samples were collected at 0.083, 0.25, 0.5, 1, 2, 4, 6, 8, and 24 hours post-dose using a staggered sampling method. Group 2: mice received an oral (gavage) dose of Compound 1 at 50 mg/kg with a dosing volume of 10 mL/kg. Blood samples were collected at 0.25, 0.5, 1, 2, 4, 6, 8, and 24 hours post-dose using a staggered sampling method. Group 3: mice received an oral (gavage) dose of palbociclib at 50 mg/kg with a dosing volume of 10 mL/kg. Blood samples were collected at 1, 3, 4, 6, 8, 10, 12, and 24 hours post-dose using a staggered sampling method. For each group, 3 samples were collected at each time point. Plasma was separated by centrifugation and stored at - 70°C. The concentrations of Compound 1 or palbociclib in plasma were measured using LC-MS/MS. The results are shown in Table 9.

**Table 9. Main Pharmacokinetic Parameters of Compound 1 in Male Balb/c Mice Following a Single Intravenous or Oral Administration**

| Administration Route | Dose (mg/kg) | C ₘₐₓ (ng/mL) | AUC ₀₋ₜ (ng·h/mL) | Tₘₐₓ (h) | T _{1/2} (h) | CL (mL/min/kg) | Vdss (L/kg) | F( %) |
|---|---|---|---|---|---|---|---|---|
| IV (Compound 1) | 5 | - | 670 | - | 2.69 | 119 | 11 | - |
| PO (Compound 1) | 50 | 21 | 39.2 | 1.0 | - | - | - | 0.6 |
| PO (palbociclib) | 50 | 1323 | 18333.3 | 8 | 7.64 | - | - | - |

As shown in Table 9, the plasma concentration of Compound 1 following oral administration is significantly lower than that of palbociclib administered orally. This indicates that the in vivo exposure of the compound described in the present application is substantially lower than that of existing CDK inhibitors.

### Example 7 Single-Dose Intravenous and Oral Pharmacokinetic Study in Rats

Six male Sprague-Dawley (SD) rats were randomly divided into two groups, with three rats in each group. Compound 1 was administered either by intravenous injection at a dose of 2 mg/kg or by oral gavage at a dose of 10 mg/kg. The dosing volumes were 5 mL/kg for intravenous administration and 10 mL/kg for oral administration, with purified water used as the solvent. For the intravenous group, blood samples were collected at 0.083, 0.25, 0.5, 1, 2, 4, 6, 8, and 24 hours post-dose. For the oral group, blood samples were collected at 0.25, 0.5, 1, 2, 4, 6, 8, and 24 hours post-dose. Plasma samples were prepared by centrifugation and stored at -70°C. The plasma concentration of Compound 1 was determined using LC-MS/MS. The results showed that, following oral administration, the plasma concentrations of Compound 1 were all below the lower limit of quantification (1 ng/mL). The main pharmacokinetic parameters are shown in Table 10.

**Table 10 Main Pharmacokinetic Parameters of Compound 1 in Male SD Rats Following Single Intravenous and Oral Administration**

| Administra tion Route | Dose (mg/kg ) | Cₘₐₓ (ng/mL) | AUC₀₋ₜ (ng·h/mL) | Tₘₐₓ (h) | T_{1/2} (h) | CL (mL/min/kg) | Vdss (L/kg) | F( %) |
|---|---|---|---|---|---|---|---|---|
| IV | 2 | - | 602±161 | - | 5.55±0.503 | 56.7±17.3 | 9.61±5.43 | - |
| PO | 10 | - | - | - | - | - | - | - |

| | | | | | | | | |
|---|---|---|---|---|---|---|---|---|
| Note: In the PO group, all plasma sample concentrations were below the lower limit of quantification (1 ng/mL); therefore, relevant pharmacokinetic parameters could not be calculated. | | | | | | | | |

### Example 8 Intestinal Tissue Distribution Study Following a Single Oral Administration in Rats

Nine male Sprague Dawley rats were orally administered Compound 1 at a dose of 25 mg/kg with a dosing volume of 10 mL/kg using purified water as the vehicle. At 0.25, 2, and 6 hours post-dosing, three animals were euthanized at each time point. A 200 µL whole blood sample was collected via cardiac puncture and plasma was separated by centrifugation.

The duodenum, jejunum, ileum, and colon were collected, contents removed, rinsed with ice-cold water, and surface moisture was blotted with filter paper before weighing. For each gram of tissue, 5 mL of methanol-water (1:3, v/v) was added for homogenization. The drug concentrations in plasma and intestinal tissue homogenates of each intestinal segment tissue were measured using LC-MS/MS.The ratios of AUC values for each intestinal segment to plasma concentration are shown in Table 11.

**Table 11 Ratios of Intestinal Tissue to Plasma Concentrations Following a Single Oral Administration in Male Rats**

| Matrix | AUC _{0-tε} ( ng·h /mL or ng·h /g) | Tissue-to-plasma concentration ratio |
|---|---|---|
| Plasma | 1.26 | / |
| Duodenum | 19300 | 15300 |
| Jejunum | 5420 | 4300 |
| Ileum | 5980 | 4750 |
| Colon | 632 | 502 |

## Claims

1. A compound of formula (I), or a pharmaceutically acceptable salt, bioactive metabolite, solvate, hydrate, prodrug, racemate, enantiomer or stereoisomer thereof, 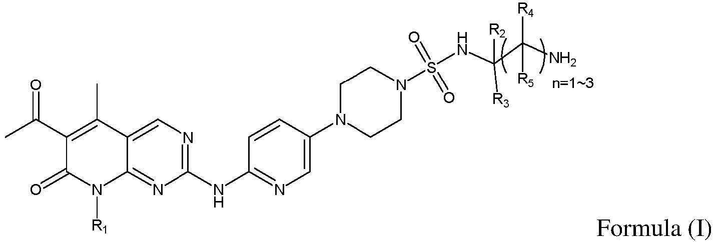 wherein,
n is 1, 2 or 3,
R1 is selected from: hydrogen, substituted or unsubstituted alkyl, substituted or unsubstituted cycloalkyl, substituted or unsubstituted alkenyl;
R₂, R₃, R₄, and R₅ are each independently selected from: hydrogen, substituted or unsubstituted alkyl, substituted or unsubstituted cycloalkyl, and substituted or unsubstituted alkenyl;
Or any two of R₂, R₃, R₄ and R₅ are joined to form a ring.

2. The compound of claim 1, or a pharmaceutically acceptable salt, bioactive metabolite, solvate, hydrate, prodrug, racemate, enantiomer or stereoisomer thereof, wherein the substitution includes heteroatom substitution.

3. The compound of any one of claims 1 to 2, or a pharmaceutically acceptable salt, bioactive metabolite, solvate, hydrate, prodrug, racemate, enantiomer or stereoisomer thereof, wherein the substitution includes alkyl, cycloalkyl or alkenyl substitution.

4. The compound of any one of claims 1 to 3, or a pharmaceutically acceptable salt, bioactive metabolite, solvate, hydrate, prodrug, racemate, enantiomer or stereoisomer thereof, wherein R₂, R₃, R₄, R₅ are each independently selected from: C₁₋₁₀ substituted or unsubstituted alkyl, C₁₋₁₀ substituted or unsubstituted cycloalkyl, C₁₋₁₀ substituted or unsubstituted alkenyl.

5. The compound of any one of claims 1 to 4, or a pharmaceutically acceptable salt, bioactive metabolite, solvate, hydrate, prodrug, racemate, enantiomer or stereoisomer thereof, wherein R₂, R₃, R₄, R₅ are each independently selected from C₁₋₅ substituted or unsubstituted alkyl. The compound of any one of claims 1 to 5, or a pharmaceutically acceptable salt, bioactive metabolite, solvate, hydrate, prodrug, racemate, enantiomer or stereoisomer thereof, wherein

6. each R₄ is the same or different.

7. The compound of any one of claims 1 to 6, or a pharmaceutically acceptable salt, a bioactive metabolite, solvate, hydrate, prodrug, racemate, enantiomer or stereoisomer thereof, wherein each R₅ is the same or different.

8. The compound of claim 1, or a pharmaceutically acceptable salt, bioactive metabolite, solvate, hydrate, prodrug, racemate, enantiomer or stereoisomer thereof, wherein any two of R₂, R₃, R₄ and R₅ are joined to form an alkane ring.

9. The compound of any one of claims 1 to 8, or a pharmaceutically acceptable salt, bioactive metabolite, solvate, hydrate, prodrug, racemate, enantiomer or stereoisomer thereof, comprising a structure selected from the group: 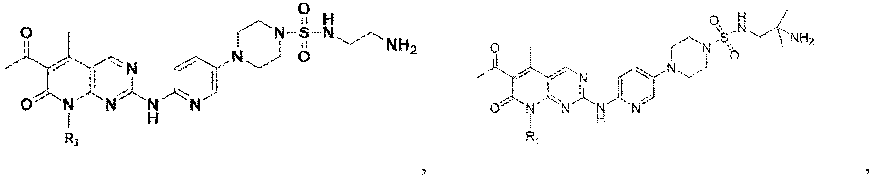 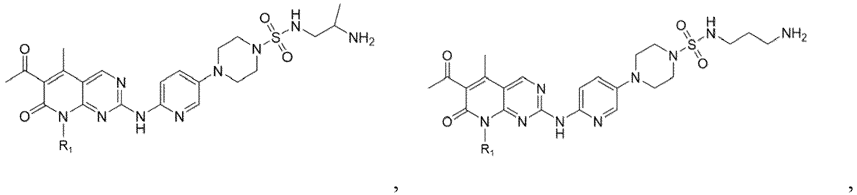 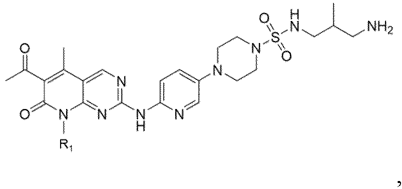 as well as

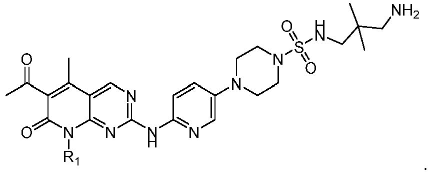

10. The compound of any one of claims 1 to 9, or a pharmaceutically acceptable salt, bioactive metabolite, solvate, hydrate, prodrug, racemate, enantiomer or stereoisomer thereof, wherein the R₁ is selected from: C₁₋₁₀ substituted or unsubstituted alkyl, C₁₋₁₀ substituted or unsubstituted cycloalkyl, C₁₋₁₀ substituted or unsubstituted alkenyl.

11. The compound of any one of claims 1 to 10, or a pharmaceutically acceptable salt, bioactive metabolite, solvate, hydrate, prodrug, racemate, enantiomer or stereoisomer thereof, wherein the R₁ is a substituted or unsubstituted cycloalkyl.

12. The compound of any one of claims 1 to **11,** or a pharmaceutically acceptable salt, bioactive metabolite, solvate, hydrate, prodrug, racemate, enantiomer or stereoisomer thereof, wherein the R₁ is a cyclopentyl.

13. The compound of any one of claims 1 to 12, or a pharmaceutically acceptable salt, bioactive metabolite, solvate, hydrate, prodrug, racemate, enantiomer or stereoisomer thereof, wherein n is 1.

14. The compound of any one of claims 1 to 12, or a pharmaceutically acceptable salt, bioactive metabolite, solvate, hydrate, prodrug, racemate, enantiomer or stereoisomer thereof, wherein n is 2.

15. The compound of any one of claims 1 to 13, or a pharmaceutically acceptable salt, bioactive metabolite, solvate, hydrate, prodrug, racemate, enantiomer or stereoisomer thereof, wherein n is 3.

16. The compound of any one of claims 1 to 15, or a pharmaceutically acceptable salt, bioactive metabolite, solvate, hydrate, prodrug, racemate, enantiomer or stereoisomer thereof, comprising a structure selected from the group:

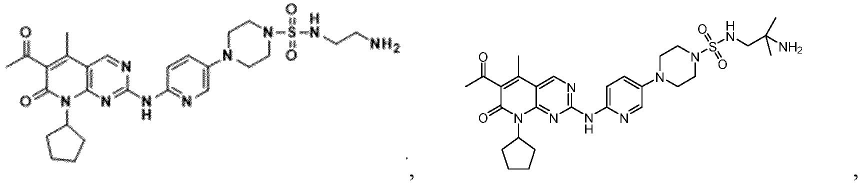

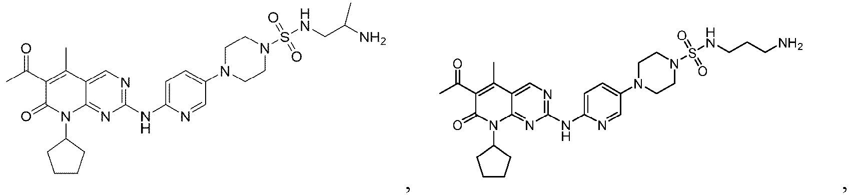

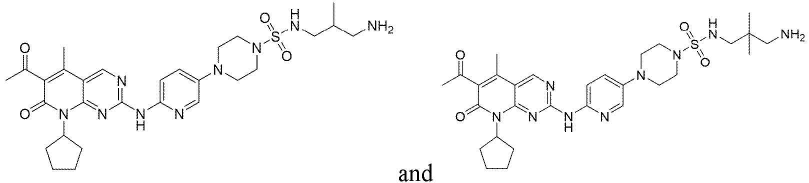

17. The compound of any one of claims 1 to 16, or a pharmaceutically acceptable salt, bioactive metabolite, solvate, hydrate, prodrug, racemate, enantiomer or stereoisomer thereof, wherein the compound is a cyclin-dependent kinase (CDK) inhibitor.

18. The compound of any one of claims 1 to 17, or a pharmaceutically acceptable salt, bioactive metabolite, solvate, hydrate, prodrug, racemate, enantiomer or stereoisomer thereof, wherein the CDK inhibitor is a CDK4 /6 inhibitor.

19. A method for preparing the compound of any one of claims 1 to 18, or a pharmaceutically acceptable salt, bioactive metabolite, solvate, hydrate, prodrug, racemate, enantiomer or stereoisomer thereof.

20. A pharmaceutical composition comprising the compound of any one of claims 1 to 18, or a pharmaceutically acceptable salt, bioactive metabolite, solvate, hydrate, prodrug, racemate, enantiomer or stereoisomer thereof.

21. The pharmaceutical composition of claim 20, further comprising optionally a pharmaceutically acceptable carrier.

22. The pharmaceutical composition of claim 21, further comprising one or more additional active ingredients.

23. The pharmaceutical composition of any one of claims 20-22, wherein the composition is an oral dosage form.

24. The use of the compound according to any one of claims 1 to 18, or a pharmaceutically acceptable salt, bioactive metabolite, solvate, hydrate, prodrug, racemate, enantiomer or stereoisomer thereof, or the composition according to any one of claims 20 to 23 in the manufacture of a medicament for preventing, alleviating and/or treating a disease and/or disorder.

25. The use according to claim 24, wherein the disease and/or disorder comprises chemotherapy-associated gastrointestinal side effects.

26. The use according to claim 25, wherein the chemotherapy comprises administration of a chemotherapeutic agent.

27. The use according to claim 26, wherein the chemotherapeutic agent is a cytotoxic agent.

28. The use according to any one of claims 26-27, wherein the chemotherapeutic agent is selected from one or more of: DNA synthesis inhibitors, RNA synthesis inhibitors, protein synthesis inhibitors, cell division inhibitors, DNA base analogs, topoisomerase inhibitors and/or telomerase synthesis inhibitors.

29. The use according to any one of claims 26 to 28, wherein the chemotherapeutic agent is selected from fluorouracil, oxaliplatin, topotecan, irinotecan, tetrahydrofolate, docetaxel, gemcitabine, carboplatin, cisplatin, etoposide, methotrexate, doxorubicin, cytarabine, vinorelbine and capecitabine, or a combination thereof.

30. The use according to any one of claims 26-29, wherein the chemotherapeutic agent is administered continuously and/or non-continuously.

31. The use according to any one of claims 25-30, wherein the chemotherapy is used in combination with one or more other therapies.

32. The use according to any one of claims 25-31, wherein the chemotherapy-associated gastrointestinal side effects comprise gastrointestinal side effects caused by chemotherapy.

33. The use according to any one of claims 25-32, wherein the chemotherapy-associated gastrointestinal side effects comprise gastrointestinal adverse events that occur or worsen after administration of the chemotherapeutic agent.

34. The use according to claim 33, wherein, in the absence of preventive or therapeutic intervention, the gastrointestinal adverse events occur or worsen at about 1 hour, about 2 hours, about 3 hours, about 4 hours, about 5 hours, about 6 hours, about 7 hours, about 8 hours, about 9 hours, about 10 hours, about 11 hours, about 12 hours, about 1 day, about 2 days, about 4 days, about 7 days, about 2 weeks, about 3 weeks, about 1 month, about 2 months, or longer after administration of the chemotherapeutic agent.

35. The use according to any one of claims 25-34, wherein the gastrointestinal side effects comprise gastric mucosal injury diseases and/or intestinal mucosal injury diseases.

36. The use according to any one of claims 25 to 35, wherein the gastrointestinal side effects comprise diarrhea, abdominal pain, nausea, vomiting, mucositis, loss of appetite, gastric ulcer, gastritis, constipation, enteritis, intestinal perforation, intestinal hemorrhage, ulcer and/or intestinal necrosis.

37. The use according to any one of claims 25-36, wherein the gastrointestinal side effects comprise diarrhea and/or constipation.

38. The use according to any one of claims 25 to 37, wherein the severity of the gastrointestinal side effects is grade 1 or higher, grade 2 or higher, grade 3 or higher, grade 4 or higher, or grade 5 in accordance with NCI-CTCAE criteria.

39. The use according to any one of claims 25-38, wherein the medicament substantially does not affect the therapeutic efficacy of the chemotherapy.

40. The use according to any one of claims 25-39 , wherein the medicament is administered about 0.5 hours, about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours or more prior to the chemotherapy.

41. The use according to any one of claims 25-40 , wherein the medicament is administered about 0.5 hours, about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours or more after the chemotherapy.

42. The use according to any one of claims 25-41, wherein the medicament is administered concurrently with the chemotherapy.

43. The use according to any one of claims 24-42, wherein the medicament is administered in a single or multiple doses.

44. The use according to any one of claims 24-43, wherein the medicament is formulated for oral administration, intravenous injection, subcutaneous injection, intraperitoneal injection and/or intramuscular injection.

45. The use according to any one of claims 24-44, wherein the medicament is formulated as a tablet and/or a capsule.

46. A method for preventing, alleviating and/or treating a disease and/or disorder in a subject, comprising administering to a subject in need thereof a compound according to any one of claims 1 to 18, or a pharmaceutically acceptable salt, bioactive metabolite, solvate, hydrate, prodrug, racemate, enantiomer or stereoisomer thereof, or the composition according to any one of claims 20 to 23.

47. The method according to claim 46, wherein the disease and/or disorder comprises chemotherapy-associated gastrointestinal side effects.

48. The method according to claim 47, wherein the chemotherapy comprises administration of a chemotherapeutic agent.

49. The method according to claim 48, wherein the chemotherapeutic agent is a cytotoxic agent.

50. The method according to any one of claims 48-49, wherein the chemotherapeutic agent is selected from one or more of: DNA synthesis inhibitors, RNA synthesis inhibitors, protein synthesis inhibitors, cell division inhibitors, DNA base analogs, topoisomerase inhibitors and/or telomerase synthesis inhibitors.

51. The method according to any one of claims 48-50, wherein the chemotherapeutic agent is selected from fluorouracil, oxaliplatin, topotecan, irinotecan, tetrahydrofolate, docetaxel, gemcitabine, carboplatin, cisplatin, etoposide, methotrexate, doxorubicin, cytarabine, vinorelbine and capecitabine, or a combination thereof.

52. The method according to any one of claims 48-51, wherein the chemotherapeutic agent is administered continuously and/or non-continuously.

53. The method according to any one of claims 47-52, wherein the chemotherapy is used in combination with one or more other therapies.

54. The method according to any one of claims 47-53, wherein the chemotherapy-associated gastrointestinal side effects comprise gastrointestinal side effects caused by chemotherapy.

55. The method according to any one of claims 47-54, wherein the chemotherapy-associated gastrointestinal side effects comprise gastrointestinal adverse events that occur or worsen after the administration of a chemotherapeutic agent.

56. The method according to claim 55, wherein, in the absence of preventive or therapeutic intervention, the gastrointestinal adverse events occur or worsen at about 1 hour, about 2 hours, about 3 hours, about 4 hours, about 5 hours, about 6 hours, about 7 hours, about 8 hours, about 9 hours, about 10 hours, about 11 hours, about 12 hours, about 1 day, about 2 days, about 4 days, about 7 days, about 2 weeks, about 3 weeks, about 1 month, about 2 months, or longer after administration of the chemotherapeutic agent.

57. The method according to any one of claims 47-56, wherein the gastrointestinal side effects comprise gastric mucosal injury diseases and/or intestinal mucosal injury diseases.

58. The method according to any one of claims 47-57, wherein the gastrointestinal side effects comprise diarrhea, abdominal pain, nausea, vomiting, mucositis, loss of appetite, gastric ulcer, gastritis, constipation, enteritis, intestinal perforation, intestinal hemorrhage, ulceration and/or intestinal necrosis.

59. The method according to any one of claims 47-58, wherein the gastrointestinal side effects comprise diarrhea and/or constipation.

60. The method according to any one of claims 47-59, wherein the severity of the gastrointestinal side effects is grade 1 or higher, grade 2 or higher, grade 3 or higher, grade 4 or higher, or grade 5 in accordance with NCI-CTCAE criteria.

61. The method according to any one of claims 47-60, wherein the compound, or a pharmaceutically acceptable salt, bioactive metabolite, solvate, hydrate, prodrug, racemate, enantiomer or stereoisomer thereof, or the composition substantially does not affect the therapeutic efficacy of the chemotherapy.

62. The method according to any one of claims 47-61, wherein the compound, or a pharmaceutically acceptable salt, bioactive metabolite, solvate, hydrate, prodrug, racemate, enantiomer or stereoisomer thereof, or the composition is administered about 0.5 hours, about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours or more prior to administration of the chemotherapy.

63. The method according to any one of claims 47-62, wherein the compound, or a pharmaceutically acceptable salt, bioactive metabolite, solvate, hydrate, prodrug, racemate, enantiomer or stereoisomer, or the composition is administered about 0.5 hours, about 1 hour, about 2 hours, about 3 hours, about 4 hours, about 5 hours, about 6 hours, about 7 hours, about 8 hours, about 9 hours, about 10 hours, about 11 hours, about 12 hours, about 13 hours, about 14 hours, about 15 hours, about 16 hours, about 17 hours, about 18 hours, about 19 hours, about 20 hours or more after administration of the chemotherapy.

64. The method according to any one of claims 47-63, wherein the compound, or a pharmaceutically acceptable salt, bioactive metabolite, solvate, hydrate, prodrug, racemate, enantiomer or stereoisomer thereof, or the composition is administered concurrently with the chemotherapy.

65. The method according to any one of claims 46-64, wherein the compound, or a pharmaceutically acceptable salt, bioactive metabolite, solvate, hydrate, prodrug, racemate, enantiomer or stereoisomer thereof, or the composition is administered in a single or multiple doses.

66. The method according to any one of claims 46-65, wherein the compound, or a pharmaceutically acceptable salt, bioactive metabolite, solvate, hydrate, prodrug, racemate, enantiomer or stereoisomer thereof, or the composition is administered at a dose of about 10-1000 mg /kg.

67. The method according to any one of claims 46-66, wherein the subject is a cancer patient.

68. A kit comprising a compound according to any one of claims 1 to 18, or a pharmaceutically acceptable salt, bioactive metabolite, solvate, hydrate, prodrug, racemate, enantiomer or stereoisomer thereof.

69. The kit according to claim 68, further comprising instructions for use of the compound, or a pharmaceutically acceptable salt, bioactive metabolite, solvate, hydrate, prodrug, racemate, enantiomer or stereoisomer thereof.

70. The kit according to any one of claims 68-69, further comprising one or more pharmaceutically acceptable carriers.
